(19) Europäisches Patentamt
European Patent Office
Office européen des brevets

(11) EP 3 037 162 A1

(12) **EUROPEAN PATENT APPLICATION**
published in accordance with Art. 153(4) EPC

(43) Date of publication:
**29.06.2016 Bulletin 2016/26**

(21) Application number: **14837269.1**

(22) Date of filing: **22.08.2014**

(51) Int Cl.:
***B01J 3/00*** (2006.01) ***B01J 23/08*** (2006.01)
***B01J 23/10*** (2006.01) ***B01J 23/22*** (2006.01)
***B01J 23/28*** (2006.01) ***B01J 23/34*** (2006.01)
***B01J 23/745*** (2006.01) ***B01J 23/75*** (2006.01)
***B01J 23/78*** (2006.01) ***B09B 3/00*** (2006.01)
***C01B 3/02*** (2006.01) ***C01B 3/40*** (2006.01)
***C02F 11/06*** (2006.01) ***C10J 3/00*** (2006.01)
***C10J 3/02*** (2006.01) ***C10J 3/46*** (2006.01)

(86) International application number:
**PCT/JP2014/071963**

(87) International publication number:
**WO 2015/025941 (26.02.2015 Gazette 2015/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **23.08.2013 JP 2013173582**

(71) Applicant: **Tohoku University**
**Sendai-shi, Miyagi 980-8577 (JP)**

(72) Inventor: **AJIRI Tadafumi**
**Sendai-shi**
**Miyagi 980-8577 (JP)**

(74) Representative: **Plougmann Vingtoft a/s**
**Rued Langgaards Vej 8**
**2300 Copenhagen S (DK)**

(54) **METHOD FOR TREATING ORGANIC MATTER IN THE PRESENCE OF WATER, CONTACT REACTION DEVICE AND SYSTEM INCLUDING SAME, AND METHOD FOR RECOVERING WASTE HEAT FROM LOW-TEMPERATURE HEAT SOURCE**

(57) An object is to provide a novel method for processing an organic substance with a catalyst under conditions in the presence of water. According to the present invention, there is provided a method of processing an organic substance under a hydrothermal condition by utilizing an oxidation-reduction cycle of a metal oxide catalyst, the method including: (i) oxidizing an organic substance with oxygen discharged from a metal oxide catalyst having an oxidized metal value so as to form a metal oxide catalyst having a reduced metal value and an oxidized organic substance; and (ii) oxidizing, simultaneously with the above (i), the metal oxide catalyst having the reduced metal value with oxygen discharged from water so as to reproduce the metal oxide catalyst having the oxidized metal value, where the metal oxide catalyst is a solid electrolyte.

Fig.12

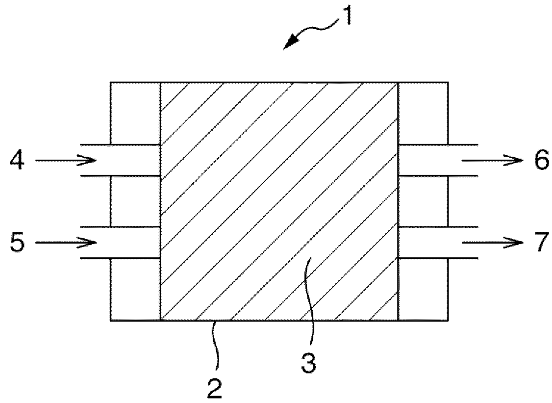


EP 3 037 162 A1

## Description

Technical Field

The present invention relates to a method of processing an organic substance in the presence of water and a device therefor. More specifically, the present invention relates to a method of utilizing the oxidation-reduction reaction of a metal oxide in the presence of water to oxidize an organic substance preferably endothermically and a device therefor.

Background Art

A large number of means for the oxidative decomposition or the lightening of an organic substance such as a hydrocarbon are conventionally proposed. Although a method of oxidatively decomposing a hydrocarbon having a relatively large molecular weight in the presence of a catalyst to lighten it attempts to be performed, there are problems in that the hydrocarbon is made heavy, coke is generated and the catalyst is deactivated thereby. Although in the presence of a hydrogenation reaction catalyst, hydrogen is introduced into a reaction system, and thus it is possible to lighten a hydrocarbon, it is disadvantageously necessary to additionally supply hydrogen whose manufacturing cost is high.

Hence, a scheme is proposed of being able to obtain a light hydrocarbon product while making a hydrocarbon ($C_nH_m$) react with water ($H_2O$) to prevent the hydrocarbon from being made heavy and the generation of coke. However, a catalyst for making such a reaction scheme effectively function is not developed. For example, Japanese Unexamined Patent Application Publication No. 2010-144094 discloses a method of obtaining tar from lignite, subjecting this tar to contact decomposition under an atmosphere of water vapor in the presence of an iron-based catalyst and thereby obtaining a hydrocarbon oil. However, the catalyst used in this method is not made to function through the oxidation-reduction of a metal oxide. Furthermore, since in this method, the raw material is specified as lignite, the efficiency is lowered due to a two-stage method and moreover, there is a limitation to reactions under an atmosphere of water vapor, industrial utilization is not practicable.

On the other hand, since in recent years, the reduction of global warming and energy saving have been increasingly intended, novel technical developments and law regulations and subsidy programs on the recovery and recycle of global waste heat (exhaust heat) are being prepared. Since waste heat is a heat source that is always constantly and stably supplied, the recovery and effective utilization thereof are required. In particular, a technology for efficiently recovering waste heat from a low-temperature heat sources is required.

As a method of recovering waste heat from a low-temperature heat source, a thermoelectric conversion element is conventionally proposed. For example, Japanese Unexamined Patent Application Publication No. 2008-78334 discloses a thermoelectric conversion element that is formed with the thermoelectric material of a crystalline oxide such as iron, cobalt or nickel. As another method of recovering waste heat from a low-temperature heat source, binary power generation is proposed. For example, Japanese Unexamined Patent Application Publication No. 6-26310 discloses a method of recovering waste heat utilizing a binary power generation turbine. However, even by means of the thermoelectric conversion element and the binary power generation, the efficiency of thermal recovery is extremely low, and it is difficult to obtain industrial and economical values.

As an alternative of the means described above, a chemical waste heat method utilizing an endothermic reaction can be considered. Examples thereof can include a method of endothermically oxidizing, in a system in the presence of water, an organic substance such as a hydrocarbon under mild conditions. In general, in an endothermic reaction in which no catalyst is used, a high temperature of 800°C or more is needed. In order to produce an endothermic reaction at a lower temperature, it is necessary to use a catalyst. In a gasification reaction at a carbonaceous high-temperature site, alkali metal catalysts such as Ca, K and Na are known. However, in such alkali metal catalysts, for example, inconveniences are produced in which the catalyst function is degraded by aggregation in the reaction, deliquescence further occurs in the presence of water vapor and dissolution in water occurs under hydrothermal conditions including a supercritical condition.

Under such conditions, the development of a novel method for endothermically oxidizing, under conditions in the presence of water, an organic substance with a catalyst is desired. The development of a method of endothermically oxidizing, under conditions in the presence of water, an organic substance with a catalyst to oxidatively decompose the organic substance while preventing a hydrocarbon from being made heavy or efficiently recovering waste heat from a low-temperature heat source is also desired. If such a reaction is possible, low-cost raw materials such as sludge, lignin, plastic waste, biomass waste, manufacturing/industrial organic waste, waste such as unused heavy hydrocarbon resource and unused resources are effectively utilized, a large amount of energy is prevented from being used for such a process and it leads to the realization of reproduction of exergy. However, such a method has not so far been proposed.

Citation List

Patent Literature

Patent Literature 1: Japanese Unexamined Patent Application Publication No. 2010-144094
Patent Literature 2: Japanese Unexamined Patent Application Publication No. 2008-78334
Patent Literature 3: Japanese Unexamined Patent Application Publication No. 6-26310

Summary of Invention

Technical Problem

Hence, an object of the present invention is to provide a novel method for processing an organic substance with a catalyst under conditions in the presence of water.

Another object of the present invention is to provide a method of endothermically oxidizing an organic substance with a catalyst under conditions in the presence of water and thereby oxidatively decomposing it while preventing a hydrocarbon from being made heavy.

Still another object of the present invention is to provide a method of using a catalyst under conditions in the presence of water and thereby efficiently recovering waste heat from a low-temperature heat source.

Yet another object of the present invention is to provide a novel device for processing an organic substance with a catalyst under conditions in the presence of water.

Solution to Problem

In order to solve the inconveniences of the conventional technology described above, the present inventor has found that it is possible to use a metal oxide catalyst which is a solid electrolyte to subject an organic substance to an oxidation reaction in the presence of water and thereby efficiently subject the organic substance to the oxidation reaction or efficiently recover waste heat from a low-temperature heat source, with the result that the present inventor completes the present invention based on the above findings.

The configurations of the present invention for solving the above problem are as follows.

[1] A method of processing an organic substance under a hydrothermal condition by utilizing an oxidation-reduction cycle of a metal oxide catalyst, the method including: (i) oxidizing an organic substance with oxygen discharged from a metal oxide catalyst having an oxidized metal value so as to form a metal oxide catalyst having a reduced metal value and an oxidized organic substance; and (ii) oxidizing, simultaneously with the above (i), the metal oxide catalyst having the reduced metal value with oxygen discharged from water so as to reproduce the metal oxide catalyst having the oxidized metal value, where the metal oxide catalyst is a solid electrolyte.

[2] The method according to [1], where in the above (i) and (ii) as a whole, $\Delta H$ (under the hydrothermal condition) $> 0$, and a reaction product obtained from the organic substance and water contains an oxidized organic substance and a hydrogen gas.

[3] The method according to [2], where the reaction product contains, in addition to the oxidized organic substance and the hydrogen gas, a substance obtained by further hydrogenating the oxidized organic substance.

[4] The method according to [1], where a reaction product obtained from the organic substance and water contains a substance obtained by further hydrogenating an oxidized organic substance.

[5] The method according to any one of [1] to [4], where in presence of supercritical water or water before criticality, reactions of the above (i) and (ii) are performed.

[6] The method according to any one of [1] to [4], where the metal oxide catalyst is selected from a group consisting of cerium oxide ($CeO_2$), indium oxide ($In_2O_3$), iron oxide ($Fe_2O_3$), yttrium-stabilized zirconium oxide (YSZ), scandium oxide-doped zirconium oxide (ScSZ), scandium oxide ($Sc_2O_3$), oxidation lanthanum gallium ($LaGaO_3$), lanthanum strontium manganite (LSM), gadolinium-doped cerium oxide (Gd-$CeO_2$), molybdenum oxide ($MoO_3$), manganese oxide ($MnO_3$), lanthanum strontium cobalt ferrite (LSCF), lanthanum strontium ferrite (LSF), tetroxide three cobalt ($Co_3O_4$), cobalt oxide II (CoO), vanadium oxide ($V_2O_5$) and ceria-zirconia solid solution.

[7] The method according to [6], where the metal oxide catalyst contains a nanoparticle of the cerium oxide ($CeO_2$).

[8] The method according to [7], where the metal oxide catalyst is formed in a shape of an octahedron and/or a cube, and contains the nanoparticle of the cerium oxide ($CeO_2$) in which a (111) plane and/or a (100) plane is a main exposure plane.

[9] The method according to any one of [1] to [8], where a large proportion of the organic substance is formed of a hydrocarbon.

[10] The method according to [9], where the above oxidation (i) includes oxidative decomposition of a hydrocarbon.

[11] The method according to any one of [1] to [8], where the organic substance contains a substance that is selected from an aldehyde, sludge, lignin, plastic waste and biomass waste.

[12] The method according to any one of [1] to [11], where the reactions of the above (i) and (ii) are performed at a temperature that is equal to or more than a room temperature but less than 450°C.

[13] The method according to any one of [1] to [12], where the above oxidation (i) includes the oxidative decomposition reaction of the hydrocarbon, and an oxidatively decomposed organic substance is further hydrogenated with hydrogen derived from water into a product that has a lower molecular weight.

[14] The method according to [13], where after the hydrogenation, a ratio of a molar yield of a saturated hydrocarbon to a molar yield of an unsaturated hydrocarbon is higher than in a decomposition reaction under no catalyst.

[15] The method according to any one of [1] to [3] and [5] to [14], where a reaction temperature is set equal to or less than 370°C, a pressure within a reaction system is set equal to or more than a saturated vapor pressure, at least a part of water is in a liquid phase and the generated hydrogen gas is separated in phase such that reaction equilibrium of the above oxidation (i) is shifted to the reaction proceeding side.

[16] A contact reaction device for processing an organic substance under a hydrothermal condition by utilizing an oxidation-reduction cycle of a metal oxide catalyst, the contact reaction device including: an introduction port of each of an organic substance and water that are reaction raw materials; a contact reactor having a reaction catalyst layer containing a metal oxide catalyst; and a discharge port of an oxidized organic substance that is a reaction product, wherein in the contact reactor, a reaction is performed that includes: (i) oxidizing an organic substance with oxygen discharged from a metal oxide catalyst having an oxidized metal value so as to form a metal oxide catalyst having a reduced metal value and an oxidized organic substance; and (ii) oxidizing, simultaneously with the above (i), the metal oxide catalyst having the reduced metal value with oxygen discharged from water so as to reproduce the metal oxide catalyst having the oxidized metal value, and the metal oxide catalyst is a solid electrolyte.

[17] The device according to [16], where in the above (i) and (ii) as a whole, $\Delta H$ (under the hydrothermal condition) > 0, and a reaction product obtained from the organic substance and water contains an oxidized organic substance and a hydrogen gas, and in the contact reaction device, the introduction ports of the organic substance and water that are the reaction raw materials are integrally formed, and the reaction catalyst layer is provided along a wall surface of a heat exchange heat transfer pipe, a waste heat fluid is passed through the heat exchange heat transfer pipe, the waste heat fluid and the reaction raw materials are brought into contact to supply heat necessary for an endothermic reaction of the organic substance and waste heat is simultaneously recovered.

[18] The device according to [17], further including: a discharge port of the hydrogen gas, where the heat exchange heat transfer pipe and the reaction catalyst layer are provided so as to form a predetermined angle with respect to a horizontal direction and the introduction ports of the organic substance and water formed integrally are provided thereabove such that the reaction on the reaction catalyst layer is performed in a method of a wet wall column.

[19] The device according to [16], where in the above (i) and (ii) as a whole, $\Delta H$ (under the hydrothermal condition) > 0, and a reaction product obtained from the organic substance and water contains an oxidized organic substance and a hydrogen gas, and in the contact reaction device, a reaction column and a catalyst flow column are included, in the catalyst flow column, a particle to which the metal oxide catalyst is carried absorbs heat from a waste heat fluid to recover heat and in the reaction column, the reaction raw materials and the particle absorbing the heat are brought into contact to supply heat necessary for an endothermic reaction of the organic substance.

[20] The device according to [16], where in the above (i) and (ii) as a whole, $\Delta H$ (under the hydrothermal condition) > 0, and a reaction product obtained from the organic substance and water contains an oxidized organic substance and a hydrogen gas, and in the contact reaction device, the introduction ports of the organic substance and water are integrally formed such that the organic substance and water are supplied as a mixture which functions both as the reaction raw materials and a waste heat fluid, and supply pipes of the organic substance and water are combined with the reaction catalyst layer to form a honeycomb type structure such that an endothermic reaction of the organic substance is performed on a wall surface of the honeycomb type structure.

[21] The device according to [20], further including: a discharge port of the hydrogen gas, where the introduction ports of the organic substance and water formed integrally are provided thereabove such that the reaction on the honeycomb type structure is performed in a method of a wet wall column.

[22] The device according to [16], where in the above (i) and (ii) as a whole, $\Delta H$ (under the hydrothermal condition) > 0, and a reaction product obtained from the organic substance and water contains an oxidized organic substance and a hydrogen gas, and in the contact reaction device, the introduction ports of the organic substance and water are integrally formed such that the organic substance and water are supplied as a mixture which functions both as the reaction raw materials and a waste heat fluid, a discharge port of the hydrogen gas is further included and the reaction catalyst layer is formed as a suspended phase that contains a particle to which the metal oxide catalyst is

carried such that while the particle is being held in the suspended phase, the oxidized organic substance and the hydrogen gas are obtained so as to be separated.

[23] The device according to [16], where in the above (i) and (ii) as a whole, ΔH (under the hydrothermal condition) > 0, and a reaction product obtained from the organic substance and water contains an oxidized organic substance and a hydrogen gas, and in the contact reaction device, a heat exchange heat transfer pipe in which a waste heat fluid and reaction raw materials are brought into contact to supply heat necessary for an endothermic reaction of the organic substance and in which waste heat is simultaneously recovered is arranged so as to make contact with the reaction catalyst layer, and another heat exchange heat transfer pipe in which after the heat exchange between the waste heat fluid and the reaction raw materials within the contact reactor, the pre-heated reaction raw materials are supplied is included.

[24] The method according to [16], where within the contact reactor, a reaction temperature is set equal to or less than 370°C, a pressure within a reaction system is set equal to or more than a saturated vapor pressure, at least a part of water is in a liquid phase and the generated hydrogen gas is separated in phase such that reaction equilibrium of the above (i) is shifted to the reaction proceeding side.

[25] A system comprising the device according to any one of [16], [17], [19], [20] and [23], the system further including: a separation chamber for separating, in an exit of the contact reactor, after cooling, a gaseous product whose main component is hydrogen gas and a product mixture solution; a recovery chamber for the gaseous product; and a recovery chamber for the product mixture solution, where based on a larger one of an amount of the gaseous product generated and passed and an amount of the product mixture solution generated and passed, pressure control for removing the product from the separation chamber to the recovery chamber is performed.

[26] A system comprising the device according to any one of [16], [17], [19], [20] and [23], where in an exit of the contact reactor, after cooling, a gaseous product and a liquid product are passed through a pipe whose inside diameter is equal to or less than 5 inches (12.7 centimeters) to constantly produce a slag flow such that a pressure of the system is stably controlled.

[27] A method of processing an organic substance under a hydrothermal condition by utilizing an oxidation-reduction cycle of a metal oxide catalyst so as to chemically recover waste heat from a low-temperature heat source, the method including: (i) oxidizing an organic substance with oxygen discharged from a metal oxide catalyst having an oxidized metal value so as to form a metal oxide catalyst having a reduced metal value and an oxidized organic substance; and (ii) oxidizing, simultaneously with the above (i), the metal oxide catalyst having the reduced metal value with oxygen discharged from water so as to reproduce the metal oxide catalyst having the oxidized metal value, where the metal oxide catalyst is a solid electrolyte, and through the oxidation-reduction cycle of the metal oxide catalyst, the waste heat is recovered as binding energy of the product which is the oxidized organic substance.

[28] The method according to [27], where in the above (i) and (ii) as a whole, ΔH (under the hydrothermal condition) > 0, and a reaction product obtained from the organic substance and water contains an oxidized organic substance and a hydrogen gas.

[29] The method according to [28], where the reaction product contains, in addition to the oxidized organic substance and the hydrogen gas, a substance obtained by further hydrogenating the oxidized organic substance.

[30] The method according to [27], where the reaction product contains, in addition to the oxidized organic substance and the hydrogen gas, a substance obtained by further hydrogenating the oxidized organic substance.

## Advantageous Effects of Invention

In the present invention, it is possible to efficiently obtain a product which is lightened while preventing the product from being heavy and coke from being formed, by subjecting an organic substance such as a hydrocarbon belonging to a wide molecular weight range to preferably an endothermic oxidation reaction in the presence of a metal oxide catalyst which is a solid electrolyte. In the present invention, it is possible to efficiently recover waste heat from a low-temperature heat source. Specifically, low-temperature waste heat and wastes which are conventionally discarded are utilized, the heat is recovered as the binding energy (enthalpy of formation) of an oxidized product, then this is burned to form a high-temperature site and thus it is possible to convert the heat of low exergy into high exergy or to directly convert it into power by a fuel battery. This is the conversion from the resources of low value to the resources of highly added value, and this can lead to the overall efficiency of an energy system. Furthermore, in an embodiment of the present invention, as a by-product, hydrogen gas derived from water molecules can be obtained, and thus it is possible to prevent the product from being made heavy, with the result that it is possible to use it as a useful supply source of hydrogen gas in the subsequent other processes.

## Brief Description of Drawings

Drawings for illustrating the present invention in a non-limiting manner will be described as follow.

[Fig. 1] A diagram showing an XRD pattern of nanoparticles of $CeO_2$ which were generated;
[Fig. 2] A diagram showing the form of the nanoparticles of $CeO_2$ which were generated;
[Fig. 3] A diagram showing an FTIR spectrum of the nanoparticles of $CeO_2$ in the shape of a cube which were generated;
[Fig. 4] Fig. 4 - a is a diagram showing a relationship between a retention time and a GC-MS spectrum strength in example 1 (comparison with the present invention: without use of a catalyst); and Fig. 4 - b is a diagram showing a relationship between a reaction time and the GC-MS spectrum strength in example 1 (the present invention: use of a catalyst);
[Fig. 5] Fig. 5 - a is a diagram showing a relationship between a reaction time and the yield of a product (acetaldehyde and acetic acid) in the case of the use and non-use of a catalyst in example 1; and Fig. 5 - b is a diagram showing a relationship between a reaction time and the yield of a product (ethanol, ethyl acetate and aldol condensation product) in the case of the use and non-use of a catalyst in example 1;
[Fig. 6] A diagram showing a reaction device and reaction conditions in example 2;
[Fig. 7] A diagram visually showing results under predetermined reaction conditions in example 2;
[Fig. 8] A diagram visually showing results under the predetermined reaction conditions in example 2;
[Fig. 9] A diagram visually showing results under the predetermined reaction conditions in example 2;
[Fig. 10] A diagram visually showing results under predetermined reaction conditions in example 3;
[Fig. 11] A diagram visually showing results under the predetermined reaction conditions in example 3;
[Fig. 12] A schematic diagram showing a contact reaction device according to the present invention;
[Fig. 13] A diagram showing an example of a heat exchange type contact reaction device according to the present invention (when waste heat is recovered as a gas or a liquid);
[Fig. 14] A diagram showing an example of a heat exchange type/wet wall type contact reaction device according to the present invention (when waste heat is recovered as a gas or a liquid);
[Fig. 15] A diagram showing an example of a two-column circulation type flow layer contact reaction device according to the present invention (when waste heat is recovered as a gas or a liquid);
[Fig. 16] A diagram showing an example of a heat exchange type contact reaction device according to the present invention (when waste heat is stored in a mixture solution of an organic substance and water which are reaction raw materials);
[Fig. 17] A diagram showing an example of a heat exchange type/wet wall type contact reaction device according to the present invention (when waste heat is stored in a mixture solution of an organic substance and water which are reaction raw materials);
[Fig. 18] A diagram showing an example of a catalyst suspension chamber type contact reaction device (when waste heat is stored in a mixture solution of an organic substance and water which are reaction raw materials);
[Fig. 19] A diagram showing the results of an OSC measurement on a metal oxide group which is a solid electrolyte;
[Fig. 20] A diagram showing OSC temperature dependence on the metal oxide group which is a solid electrolyte;
[Fig. 21] A diagram showing the appearance of a pigment (indigo carmine) aqueous solution after a low temperature oxidation reaction is performed in the presence of various types of metal oxide catalysts in example 4; and
[Fig. 22] A diagram showing the yields of acetaldehyde and acetic acid when the oxidation reaction of acetaldehyde is performed in the presence of various types of metal oxide catalysts in example 5.

Description of Embodiments

Water used in the reaction of the present invention may be supercritical water (SCW) or may be water before criticality. The water before criticality includes water in a state that is referred to as water in a gas phase or water vapor (or steam). The water before criticality includes water in a state that is referred to as subcritical water. In the case of the water before criticality, water in a liquid state (in a liquid phase) or a liquid phase is preferably included as a main phase. Under such hydrothermal conditions, an ability to form a single phase together with a relatively heavy hydrocarbon is acquired, and it is possible to significantly control, in the vicinity of a critical point, solvent effects (influences given to reaction equilibrium/speed accompanied by a dielectric constant and the formation of a hydrated structure) by a temperature and a pressure. In an embodiment, in a hydrogen gas produced by the generation of the reaction of the present invention, phase separation occurs in a subcritical region, and it is possible to significantly control a reduction reaction in the vicinity of a critical point. Hence, when as an organic substance of a raw material, a relatively heavy hydrocarbon is used, subcritical water or supercritical water is preferably used. With consideration given to the separation of a gas phase, subcritical water can be selected.
The oxidation reaction of an organic substance in the present invention is assumed to include the addition reaction of normal oxygen to an organic substance and an oxidative decomposition reaction of an organic substance (reaction in which constituent molecules are divided or cleaved into smaller molecules while being coupled to oxygen atoms).

The "hydrothermal conditions" in the present invention are defined as conditions in the presence of water having the following reaction temperatures. The "hydrothermal conditions" herein include, as described above, conditions in the presence of water in a state that is referred to as water in a gas phase or water vapor (or steam). As the reaction temperature of the present invention, different temperatures can be adopted depending on the type of organic substance used as the raw material and the composition of a target product. The reaction temperature is not particularly limited but is normally equal to or more than room temperature (15 to 30°C) and may be equal to or less than 1000°C. The reaction temperature may be more typically equal to or more than 15°C but equal to or less than 600°C, may be preferably equal to or more than 25°C but equal to or less than 500°C and may be more preferably equal to or more than 30°C but less than 450°C. In particular, when the organic substance is a substance that is selected from an aldehyde, sludge, lignin, plastic waste and biomass waste, in terms of efficiently recovering waste heat from a low-temperature heat source, the reaction temperature may be preferably less than 450°C, and may be more preferably equal to or less than 400°C. Since in the oxidation reaction of formaldehyde, the raw material has a low molecular weight, a reaction around the room temperature is anticipated. Detoxification produced by the oxidation of formaldehyde is effective as a measure for sick building syndrome.

Reaction pressure in the present invention is not particularly limited but may be equal to or more than the atmospheric pressure but equal to or less than 50 MPa. The reaction pressure may be more preferably equal to or more than 5 MPa but equal to or less than 40 MPa.

Reaction time in the present invention is not particularly limited but for example, may be equal to or more than 1 minute but equal to or less than 48 hours, may be more generally equal to or more than 5 minutes but equal to or less than 24 hours and may be more typically equal to or more than 10 minutes but equal to or less than 12 hours.

The organic substance used in the present invention is not particularly limited as long as a large proportion thereof is formed of a carbon-containing compound. A typical carbon-containing compound is a hydrocarbon (a single type of hydrocarbon having a saturated bond and in some cases, an unsaturated bond or a mixture of a plurality of types). As the organic substance that can be used, bitumen (including, for example, marten and asphaltene) or asphalt, which is an extremely important hydrocarbon, is included. In an embodiment, bitumen or asphalt, which is an extremely important hydrocarbon, may be prevented from being used as the organic substance of the present invention with consideration given to the efficiency of industrial practice. The organic substance may contain at least one type selected from a group of an aldehyde (formaldehyde and/or acetaldehyde), sludge, lignin, plastic waste (industrial wastes from a company and/or a home) and biomass waste. As the organic substance, manufacturing/industrial organic waste and unused heavy hydrocarbon resources are also included. The biomass waste is not particularly limited but examples thereof can include paper, livestock manure, food waste, construction waste, black liquor, sewage sludge, garbage, rice straw, wheat straw, chaff, forest remainder (such as thinning material and damaged trees), resource crop, forage crop and starch-based crop etc.

The reaction of the present invention utilizes the oxidation-reduction cycle of a metal oxide catalyst. The metal oxide catalyst is preferably a solid electrolyte. The metal oxide catalyst typically makes it possible to satisfy, both in the reactions of the above (i) and (ii), $\Delta G < 0$ which is calculated under hydrothermal conditions by utilizing a HKF mode. In the metal oxide catalyst which is a solid electrolyte, typically, an oxygen storage capacity (hereinafter abbreviated as an "OSC") at a temperature equal to or more than room temperature but equal to or less than 450°C is 1 $\mu$ mol - O/g - cat (the number of moles of oxygen per gram of the catalyst) or more. More preferably, the OSC at a temperature equal to or more than a room temperature but equal to or less than 450°C may be 10 $\mu$ mol - O/g - cat or more.

As an example, a reaction scheme in an embodiment of the present invention when as the metal oxide catalyst, cerium oxide ($CeO_2$) is used, and as the organic substance, a hydrocarbon (schematically represented as HC) is used is as follows.

$$CeO_2 + HC \rightarrow Ce_2O_3 + HC(O)$$

$$H_2O + Ce_2O_3 \rightarrow 2CeO_2 + H_2$$

As a whole,

$$HC + H_2O \rightarrow HC(O) + H_2 \text{ (gas)}$$

As described above, in the endothermic oxidation reaction scheme according to the embodiment of the present invention, through the oxidation-reduction of the metal oxide catalyst, hydrocarbons and water are made to react with each other, and thus the oxidized hydrocarbon and hydrogen gas are generated. In the entire reaction, $\Delta H$ (T, P under reaction conditions) > 0 holds true. It is known that an oxidation-reduction reaction accompanied by the generation of hydrogen gas is normally an endothermic reaction.

A reaction scheme in another embodiment of the present invention is as follows. In the reaction scheme, a

substance in which the oxidized hydrocarbon is further hydrogenated is obtained.

$$CeO_2 + HC \rightarrow Ce_2O_3 + HC(O)$$

$$H_2O + Ce_2O_3 \rightarrow 2CeO_2 + H_2 \text{ (gas)}$$

$$HC(O) + H_2 \rightarrow HC(O)\ (H)$$

In still another embodiment of the present invention, without being accompanied by the substantial occurrence of hydrogen gas, the substance in which the oxidized hydrocarbon is further hydrogenated may be obtained. Although it is not intended to be theoretically limited, it can be considered that hydrogen left on the catalyst in the form of a hydrogen molecule ($H_2$) or a hydrogen ion ($H^+$) or a hydrogen radical ($H\cdot$) is taken in the oxidized hydrocarbon, and thus without being accompanied by the substantial occurrence of hydrogen gas (without hydrogen gas being discharged to the outside of the system), the oxidized and hydrogenated hydrocarbon can be obtained as a product. In this case, it is possible that the entire reaction up to the formation of the product is not an endothermic reaction ($\Delta H$ (T, P under reaction conditions) > 0).

A HKF model is named from an abbreviation for three researches, that is, Helgeson, Kirkham and Flowers, and is known as a semi-theoretical formula for the evaluation of solvent effects (influences of a temperature, a pressure, a density and a dielectric constant) given to reaction equilibrium in high-temperature water. $\Delta G$: Gibbs energy difference in a reaction is calculated as a value obtained by subtracting the total of the chemical potentials ($\mu$) of reaction raw materials from the total of the chemical potential ($\mu$) of a product. The correction for the temperature of the chemical potential ($\mu$) is performed by a calculation formula of $\mu_i = \mu_{iO} + \Delta h_i (T) - T\Delta S_i (T)$. The correction for the pressure of the chemical potential ($\mu$) is performed by $K = P_{H2} / P_{H2O} = \exp(-(\mu_{H2} - \mu_{H2O}) / RT) = Ko \exp(-\Delta H / RT)$ (ideal gas conditions). The chemical potential of each substance i hydrated under hydrothermal conditions including a supercritical condition is determined by a calculation formula of $\mu_i (T, \rho, \varepsilon) = \mu_{i0} + \Delta_{\mu i} (T, \rho, \varepsilon)$, using the HKF model. As described above, by the introduction of the HKF model, it is possible to evaluate reaction conditions (T, P, a water density $\rho$ and a dielectric constant $\varepsilon$) in which under hydrothermal conditions including a supercritical region, the two elementary reactions described above are $\Delta G < 0$ and which are more likely to proceed in terms of the equilibrium theory and a metal oxide group.

In another embodiment of the present invention, the reaction temperature may be equal to or less than 370°C, the pressure within the reaction system may be equal to or more than a saturated vapor pressure and at least part of water may be in a liquid phase. In this case, the generated hydrogen gas is separated in phase and is discharged as bubbles to the outside of the reaction system. The generated hydrogen gas is separated in phase in this way, and thus it is possible to shift the reaction equilibrium of the oxidization of the organic substance to the reaction proceeding side. In such an aspect, surprisingly, even when $\Delta G > 0$, it is industrially advantageous in that it is possible to make the oxidation reaction of the organic substance proceed.

The OSC of the metal oxide used in the present invention at a temperature equal to or more than a room temperature but equal to or less than 450°C is normally 1 $\mu$ mol - $O_2$/g - cat (the number of moles of oxygen per gram of the catalyst) or more. It is generally known that the OSC of a metal oxide is increased as the temperature is increased. The OSC of the metal oxide used in the present invention may be preferably 10 $\mu$ mol - $O_2$/g - cat (the number of moles of oxygen per gram of the catalyst) or more, may be more preferably 15 $\mu$ mol - $O_2$/g - cat (the number of moles of oxygen per gram of the catalyst) or more and may be further preferably 20 $\mu$ mol - $O_2$/g - cat (the number of moles of oxygen per gram of the catalyst) or more.

A method of measuring the oxygen storage capacity (OSC) is not particularly limited, and any one of known methods may be used. A specific example of the measurement method is as follows.

[Method example 1]

A gas adsorption device is used, a catalyst sample is set on a measurement cell and its temperature is increased to a predetermined temperature. Then, $H_2$ gas is introduced at a predetermined secondary pressure to perform reduction for 900 seconds. The $H_2$ gas is replaced with He for 300 seconds, $O_2$ gas measured with a measuring tube of 1 $cm^3$ is introduced by pulses into a carrier gas of He and it is detected with a TCD. When the sample absorbs $O_2$, the amount of $O_2$ in the carrier gas is reduced. Until the reduction is stopped, the introduction by pulses is repeated, and the total of the amount of $O_2$ gas reduced can be set to the oxygen storage capacity. In part of the example disclosed in the present specification, the sample is exposed to $O_2$ and He every 20 minutes, and thus the oxygen storage capacity (OSC) is measured.

More specifically, the method example 1 described above is performed as follows. The gas adsorption device is used, the catalyst sample is set on the measurement cell and then, the temperature of the catalyst sample is increased to a predetermined temperature of 250 to 500°C while He gas is being introduced at the predetermined secondary

pressure (an ordinary pressure or about a pressure equal to or more than 1 atmosphere but equal to or less than 3 atmospheres). Then, $O_2$ 5% gas / He 95% mixture gas (carrier gas) obtained by mixing 5% of $O_2$ gas with the He gas is introduced into the He gas, CO 4% gas / He 96% mixture gas is introduced into the carrier gas by pulses and analysis is performed by MS (Mass Spectrometry). When the sample absorbs $O_2$, the amount of $O_2$ in the carrier gas is reduced. Until the reduction is stopped, the introduction by pulses is repeated, and the total of the amount of $O_2$ gas reduced can be set to the oxygen storage capacity.

[Method example 2]

Another example of the method of measuring the OSC is as follows.

1. He gas is passed into the measurement system at 500°C.
2. $O_2$ gas is passed into the measurement system at 500°C, and a sufficient amount is adsorbed to the sample.
3. He gas is passed into the measurement system at 500°C.
4. $H_2$ gas is passed into the measurement system at 500°C, and the sample is reduced to remove the absorbed $O_2$.
5. He gas is passed into the measurement system at 500°C.
6. He gas is passed into the measurement system at a detection temperature (350°C) (the processing described above is preprocessing).
7. He gas serving as the carrier gas is passed and $O_2$ gas is passed by pulses into the measurement system at a detection temperature (350°C).
8. Until the $O_2$ gas of pulses passed is detected with a detector, $O_2$ gas is passed by pulses into the measurement system.
9. A value obtained by subtracting the total detected amount from the total amount of $O_2$ gas flowing out is estimated as the total absorbed amount ($cm^3$).
10. A unit absorbed amount ($cm^3/g$) is calculated from the total absorbed amount ($cm^3$) determined in the above 9 and a prepared amount (g).

For another part of the example disclosed in the present specification, this method is used to measure the OSC.

The metal oxide used in the present invention preferably has, to some extent, a low solubility in an aqueous solution. The solubility of the metal oxide in water of room temperature may be generally equal to or less than 10 g / 1 kg, may be typically equal to or less than 8 g / 1 kg and may be preferably equal to or less than 5 g / 1 kg.

In order to have an oxygen mobility necessary for an oxidation-reduction reaction, it is preferable to use, as the catalyst in the processing method of the present invention, a metal oxide used as the solid electrolyte of a fuel battery. Non-limiting examples of the metal oxide of the solid electrolyte described above include cerium oxide ($CeO_2$), indium oxide ($In_2O_3$), iron oxide ($Fe_2O_3$), yttrium-stabilized zirconium oxide (YSZ), scandium oxide-doped zirconium oxide (ScSZ : also referred to as ScZ), scandium oxide ($Sc_2O_3$), oxidation lanthanum gallium ($LaGaO_3$), lanthanum strontium manganite (LSM), gadolinium-doped cerium oxide (Gd-$CeO_2$), molybdenum oxide ($MoO_3$), manganese oxide ($MnO_3$), lanthanum strontium cobalt ferrite (LSCF), lanthanum strontium ferrite (LSF), tetroxide three cobalt ($Co_3O_4$), cobalt oxide II (CoO), vanadium oxide ($V_2O_5$) and ceria-zirconia solid solution. Among them, cerium oxide ($CeO_2$), indium oxide ($In_2O_3$), iron oxide ($Fe_2O_3$), yttrium-stabilized zirconium oxide (YSZ), scandium oxide-doped zirconium oxide (ScSZ), scandium oxide ($Sc_2O_3$), oxidation lanthanum gallium ($LaGaO_3$), lanthanum strontium manganite (LSM), gadolinium-doped cerium oxide (Gd-$CeO_2$), cobalt oxide II (CoO) and vanadium oxide ($V_2O_5$) are preferable, and cerium oxide ($CeO_2$) is more preferable. The metal oxide catalysts described above may be used singularly or a plurality of types thereof may be used by being mixed.

The particle diameter and the form of the metal oxide catalyst is not particularly limited but in terms of maximizing the surface area of the exposure to a reactant (the possibility of contact) and maximizing the realization of the catalyst function, the average particle diameter is preferably equal to or less than a few micrometers and a nanoparticle catalyst (catalyst whose average particle diameter is on the order of nanometers (less than 1 $\mu$m)) is particularly preferable. The average particle diameter is not particularly limited but the lower limit may be generally equal to or more than 2 nm, may be more generally equal to or more than 5 nm and may be more typically equal to or more than 10 nm. The upper limit may be generally equal to or less than 5 $\mu$m, may be more generally equal to or less than 1 $\mu$m, may be typically equal to or less than 500 nm, may be more typically equal to or less than 300 nm, may be more preferably equal to or less than 200 nm and may be more preferably equal to or less than 100 nm. Although the form of the metal oxide catalyst may be spherical or pseudo-spherical or may be the shape of a polyhedron, the form of the particles that can be achieved is dependent on the type of the metal oxide and the manufacturing conditions. In terms of the reaction activity, the specific surface area of the metal oxide catalyst may be equal to or more than 5 $m^2/g$ but equal to or less than 1000 $m^2/g$, may be more generally equal to or more than 10 $m^2/g$ but equal to or less than 500 $m^2/g$, may be typically equal to or more than 20 $m^2/g$ but equal to or less than 400 $m^2/g$ and may be preferably equal to or more than 30 $m^2/g$ but equal to or

less than 300 $m^2/g$.

The nanoparticle catalyst of $CeO_2$ can be formed in the shape of an octahedron or a cube. Here, the nanoparticle catalyst of $CeO_2$ has a (111) plane and/or a (100) plane as the main exposure plane. The (100) plane of $CeO_2$ is unstable and has higher oxygen mobility (oxygen storage release capacity), and thus it is possible to obtain a higher catalytic activity. Hence, in the present invention, the nanoparticle catalyst of $CeO_2$ in the shape of a cube is preferably used.

It is clarified that in the nanostructure of $CeO_2$, six (100) planes have the largest surface energy among the crystal planes of low index. The high surface energy is caused by the instability of oxygen of the top layer which is a cross-linking position between cerium ions. It can be considered that a high inversion rate of the organic substance is achieved by the instability of oxygen. The oxygen of the top layer of $CeO_2$ in the shape of a cube is discharged depending on the temperature and pressure. The oxygen species is moved to a reactant, and it can be decomposed into a product. Ce in the state of a valence of + 4 is inverted into Ce in the state of a valence of + 3 so as to become instable. A vacancy of ceria oxygen is produced by $Ce^{3+}$, and the vacancy formed in the reduced ceria surface causes a reaction with water molecules, and is coupled to oxygen into Ce in the state of a valence of + 4. In some cases, the hydrogen molecules discharged may be moved to a decomposition compound to cause a hydrogenation reaction.

Although the reaction method of the present invention may be a batch method or a continuous method, the continuous method is more preferably used. The reaction of the present invention can be performed, at least in lab scale, with a plug flow reactor. The amount of water supplied in the reaction of the present invention may be adjusted based on the organic substance introduced into the reaction system such that for example, a molar ratio of 0.01 to 100 is retained. The amount of metal oxide catalyst used is not particularly limited but for example, based on the volume of the reactor used, the amount of metal oxide catalyst packed may be equal to or more than 0.05 volume % but equal to or less than 70 volume %, and may be more typically equal to or more than 5 volume % but equal to or less than 60 volume % by formation of a catalyst-packed layer.

The metal oxide nanoparticles used in the method of the present invention are not particularly limited but for example, they can be manufactured by a method disclosed in Japanese Patent No. 3047110 (where one of the inventors is the present inventor).

The literature discloses that the aqueous solution of a metal salt (metal salt of IB group metal, IIA group metal, IIB group metal, IIIA group metal, IIIB group metal, IVA group metal, IVB group metal, VA group metal, VB group metal, VIB group metal, VIIB group metal, transition metal or the like) is continuously supplied to a distribution type reactor in a reaction range of conditions of subcritical to supercritical water in which the temperature is equal to or more than 200°C and the pressure is equal to or more than 160 $km/cm^2$, and a reducing gas (for example, hydrogen) or an oxidizing gases (for example, oxygen) is introduced into the aqueous solution of the metal salt to manufacture metal oxide fine particles.

As an example of another method of manufacturing fine particles, a method is disclosed in Japanese Patent No. 3663408 (where one of the inventors is the inventor of the present application).

The literature discloses a method of manufacturing fine particles using high-temperature and high-pressure water in which water is passed through a pressuring means and a heating means to form high-temperature and high-pressure water in a supercritical state or a subcritical state, a fluid raw material is cooled to a temperature lower than the critical temperature of water before being merged with the high-temperature and high-pressure water, then the high-temperature and high-pressure water is merged with the fluid raw material at a mixing portion and thereafter they are guided to a reactor.

The metal oxide nanoparticles used in the method of the present invention are not particularly limited but for example, it is possible to recover and collect them after manufacturing by a method disclosed in Japanese Patent No. 3925936 (where one of the inventors is the inventor of the present application).

In the method disclosed in the literature,

(i) high-temperature and high-pressure water is used as a reaction site, and a metal compound is subjected to a hydrothermal reaction to form the metal oxide nanoparticles of $CeO_2$ or the like,
(ii) high-temperature and high-pressure water is used as the reaction site, a surface of the metal oxide nanoparticles and an organic modifier are made to reach with each other, a hydrocarbon group which may be replaced or may not be replaced is bound to the surface of the nanoparticles through a bond selected from a group consisting of a covalent bond, an ether bond, an ester bond, a bond through an N atom, a bond through an S atom, a bond of metal-C-, a bond of metal-C= and a bond of a metal-(C=O)- and the surface of the nanoparticles is organically modified,
(iii) the metal oxide nanoparticles are obtained by (1) precipitating and recovering the metal oxide nanoparticles which are dispersed in an aqueous solution, (2) transferring the metal oxide nanoparticles which are dispersed in an aqueous solution into an organic solvent and recovering them or (3) collecting the metal oxide nanoparticles on an organic solvent phase-water phase interface.

The synthesis of the nanoparticles of $CeO_2$ which are a typical metal oxide catalyst will be described below.

The nanoparticles of $CeO_2$ in the shape of an octahedron can be synthesized by a known method.

The nanoparticles of $CeO_2$ in the shape of a cube can be synthesized by a method that includes (1) preparing a raw material solution in toluene, (2) using an organic modifier to synthesize the nanoparticles of $CeO_2$ in the shape of a cube under conditions of supercritical water and (3) removing the organic modifier without changing the form of $CeO_2$ in the shape of a cube.

Specifically, the preparation of the nanoparticles of $CeO_2$ in the shape of a cube can be performed as follows. This is a non-limiting example.

In toluene, as an organic modifier, hexane acid and $Ce(OH)_4$ are dissolved, and thus a nanoparticle precursor solution of cerium oxide in the shape of a cube is prepared. Thereafter, in order to obtain the clear solution, the precursor solution is mixed while being continuously agitated. The precursor solution is mixed with deionized water, and a furnace is used to rapidly increase the precursor solution to a temperature of 600 to 700 K. Then, the mixture is cooled. The nanoparticles of cerium oxide in the shape of a cube can be obtained as a dispersion product in the mixture of water, toluene and an unreacted raw material. Ethanol is added to the nanoparticles in the phase of toluene, the mixture is purified by centrifugation and decanting and thus the unreacted organic molecules are removed. The particles are dispersed in cyclohexane and are thereafter freeze dried under vacuum. In order for any organic ligand to be removed from the surface of the particles, the collected nanoparticles are calcined in air for a few hours at a high temperature of about 300°C. The calcined nanoparticles are purified by centrifugation and decanting and are then dried under reduced pressure, with the result that the nanoparticles of $CeO_2$ in the shape of a cube can be obtained.

In the reaction of the present invention, the oxidized organic substance which is the product may be further hydrogenated by hydrogen derived from water. This embodiment includes a case where without being accompanied by the substantial occurrence of hydrogen gas, the substance in which the oxidized organic substance is further hydrogenated is obtained. Although it is not theoretically limited, this reaction is understood as a phenomenon in which for example, at a relatively high temperature (for example, equal to or more than 250°C but equal to or less than 500°C) and/or under conditions in which the hydrogen concentration is high, hydrogen derived from water (hydrogen molecule, hydrogen ion or hydrogen radical) is consumed by the free alkyl chain molecule of the organic substance and thus lighter molecules are formed. This reaction includes, for example, a reaction in which hydrogen reacts with olefin produced by the oxidative decomposition of the organic substance to obtain alkane. As a desirable result, after the hydrogenation, the molar yield of alkane (except methane) may often be higher than the molar yield of alkene. By the reaction described above, it is additionally advantageous that it is possible to further reduce an increase in the molecular weight of the product or the formation of coke. When the oxidized and hydrogenated hydrocarbon is obtained as the product without being accompanied by the substantial occurrence of hydrogen gas, it is possible that the reaction as a whole is not an endothermic reaction (it is estimated that $\Delta H$ is equal to or less than 0).

In the reaction of the present invention, after the hydrogenation, a ratio of the molar yield of the saturated hydrocarbons to the molar yield of the unsaturated hydrocarbons is preferably higher than that in a decomposition reaction under no catalyst. After the hydrogenation, a ratio of the molar yield of alkane (except methane) to the molar yield of alkene is also preferably higher than that in a decomposition reaction under no catalyst.

From another point of view, the method of the present invention is utilized as a method of chemically recovering waste heat from a low-temperature heat source by preferably endothermically oxidizing an organic substance. In this method, while low-cost raw materials such as waste such as sludge, lignin, plastic waste and biomass waste and an unused resource are being effectively utilized, it is possible to realize the reproduction of exergy. Specifically, low-temperature waste heat and waste which are conventionally discarded are utilized, the heat is recovered as the binding energy (enthalpy of formation) of the oxidized product, then this is burned to form a high-temperature site and thus it is possible to convert the heat of low exergy into high exergy or to directly convert it into power by a fuel battery.

According to still another aspect of the present invention, a contact reaction device for performing the method described above is provided.

In an embodiment, this contact reaction device includes the introduction port of each of an organic substance and water which are reaction raw materials, a contact reactor and a discharge port of the oxidized organic substance which is a reaction product (and a discharge port of hydrogen gas as necessary). Fig. 12 shows a schematic diagram of the contact reaction device according to the embodiment of the present invention. The shapes and materials of the introduction port, the contact reactor and the discharge port are not particularly limited as long as it is possible to introduce and discharge predetermined amounts of reaction raw materials and product described above. Reference signs in the figure indicate the following meanings. 1: contact reaction device, 2: contact reactor, 3: region including a reaction catalyst layer containing a metal oxide catalyst, 4: introduction port of the organic substance, 5: introduction port of water (4 and 5 can be integral), 6: discharge port of the oxidized organic substance, 7: discharge port of hydrogen gas (6 and 7 can be integral)

The contact reactor included in this device is not particularly limited as long as the contact reactor includes a reaction catalyst layer containing one or a plurality of types of metal oxide catalysts described above and a desired reaction (preferably, an endothermic reaction) can be performed. Although the contact reactor may be either a batch

type reactor or a continuous type reactor, in terms of the efficiency of the reaction, the latter is preferable.

In the reaction catalyst layer of the contact reactor, a heat exchange heat transfer pipe may be provided. A waste heat fluid is passed through the heat exchange heat transfer pipe, and the waste heat fluid is brought into contact with the reaction raw materials (including the organic substance and water) to supply heat necessary for the endothermic reaction of the organic substance, with the result that it is possible to perform waste heat recovery at the same time. The waste heat fluid has heat which is normally discharged, and is not particularly limited as long as the waste heat fluid can supply heat necessary for the endothermic reaction of the organic substance.

The contact reactor may further include another heat exchange heat transfer pipe. After heat exchange is performed between the waste heat fluid and the reaction raw materials, the pre-heated reaction raw materials can be supplied to such a heat exchange heat transfer pipe.

An example of a specific structure of the contact reaction device according to the present invention is as follows.

[1]. Heat exchange type contact reaction device (in a case where waste heat is recovered as a gas or a liquid)
A specific example of the contact reaction device according to the present invention is shown in Fig. 13.
Reference signs in the figure indicate the following meanings. 1: contact reaction device, 8: reaction catalyst layer, 9: organic substance and water serving as reaction raw materials, 10: oxidized organic substance, water and hydrogen gas, 11: waste heat fluid, 12: fluid of waste heat recovery
In the figure, the introduction ports of the organic substance and water (mixture) 9 serving as the reaction raw materials are formed integrally. Black arrows in the figure mean the direction of heat transfer.
In many cases such as steel slag and a combustion exhaust gas, waste heat is recovered as a gas. However, in the case of low-temperature waste heat of mainly a boiling point or less (in many cases, water whose boiling point is 100°C or less), waste heat may be recovered as a liquid. Even in a distillation column, an extraction column and the like, there is a type of waste heat that is recovered as a liquid. Waste heat from a high-pressure process may be a high-temperature liquid. In such a case, it is necessary to perform heat exchange from the waste heat of a recovered gas (liquid) to perform a reaction.
Hence, the contact reaction device 1 that includes a heat exchange system in which the reaction catalyst layer 8 is formed along the wall surface of a heat transfer pipe is effective. The reaction raw material 9 absorbs heat through the heat transfer wall surface, this heat causes the production of an oxidation reaction (reforming reaction) and at an exit, the oxidized organic substance (reforming raw material) and hydrogen are recovered as the product 10.
When the pressure is set higher than the vapor pressure of water at the maximum reaction temperature, the heat transfer wall surface is brought into a so-called boiling heat transfer state, and a gas-liquid two-phase flow is produced. Since hydrogen gas which is the product is discharged in the gas phase, the reaction equilibrium is shifted to the side of the product. Hence, the limitation of the reaction equilibrium which is generally disadvantageous at a low temperature is removed, and thus the reaction proceeds even at a low temperature.
[2]. Heat exchange type/wet wall type contact reaction device (in a case where waste heat is recovered as a gas or a liquid)
A specific example (a variation of the above [1]) of the contact reaction device according to the present invention is shown in Fig. 14.
Reference signs in the figure indicate the following meanings. 1: contact reaction device, 8: reaction catalyst layer, 9: organic substance and water serving as reaction raw materials, 10: oxidized organic substance and water, 11: waste heat fluid, 12: fluid of waste heat recovery, 13: hydrogen gas
In the figure, the introduction ports of the organic substance and water (mixture) 9 serving as the reaction raw materials are formed integrally. A discharge port of the hydrogen gas 13 other than the discharge port of the oxidized organic substance and water 10 is provided. Black arrows in the figure mean the direction of heat transfer.
The heat exchange heat transfer pipe and the reaction catalyst layer 8 are provided so as to form a predetermined angle (preferably in the vertical direction) with respect to the horizontal direction, the introduction ports of the organic substance and the water 9 formed integrally are provided thereabove and thus the reaction on the reaction catalyst layer 8 is performed in the method of a wet wall column. In this example, the heat exchange system is formed as a vertical type, the reaction side is used as the wet wall column and thus it is possible to prevent a reduction in the reaction caused by the production of a hydrogen gas adsorption/gas reservoir in the reaction catalyst layer 8. In this case, the reaction raw materials 9 are lowered along the wall of the reaction catalyst layer 8 and is recovered from below, and the generated hydrogen gas 13 is raised in the pipe and is recovered from above. The gas of the waste heat fluid 11 on the high-temperature side is passed along an inner pipe, and thus it is possible to reduce heat loss to the outside.
Even in this case, when the pressure is set higher than the vapor pressure of water at the reaction temperature, the heat transfer wall surface of the reaction catalyst layer is brought into the so-called boiling heat transfer state, and a gas-liquid two-phase flow is produced. Since hydrogen gas which is the product is discharged in the gas phase, the reaction equilibrium is shifted to the side of the product. Hence, the limitation of the reaction equilibrium which

is generally disadvantageous at a low temperature is removed, and thus the reaction proceeds even at a low temperature.

[3]. Two-column circulation type flow layer contact reaction device (in a case where waste heat is recovered as a gas or a liquid)

A specific example of the contact reaction device according to the present invention is shown in Fig. 15.

Reference signs in the figure indicate the following meanings. 1: contact reaction device, 9: organic substance and water serving as reaction raw materials, 10: oxidized organic substance, water and hydrogen gas, 11: waste heat fluid, 12: fluid of waste heat recovery, R: reaction column, C: catalyst flow column, m: particles to which metal oxide catalyst is carried

The contact reaction device 1 of this example includes a reaction column R and a catalyst flow column C. In the catalyst flow column C, particles m to which the metal oxide catalyst is carried absorb heat from the waste heat fluid 11 to recover the waste heat whereas in the reaction column R, the reaction raw materials 9 are brought into contact with the particles m absorbing the heat to supply the heat necessary for the endothermic reaction of the organic substance. In other words, in the two-column circulation type flow layer contact reaction device 1, the articles m to which the metal oxide catalyst is carried act as a flow medium. When viscous liquid or slurry is the reaction raw material, in the heat exchange type contact reaction device of the above [1] or [2], solid precipitation or the like on a scaling or a wall surface is often problematic. In such a case, the two-column circulation type flow layer contact reaction device is effective.

In the two-column circulation type flow layer contact reaction device, the particles (m) absorbing the heat of the waste gas (liquid) are moved to the reaction column R. Since within the flow layer, the heat transfer speed is determined by the movement speed of the particles, in general, it is possible to obtain an effective heat conduction higher than the heat conductivity of a metal. In the flow layer, the particles are completely mixed, and thus the temperatures of both columns are substantially uniform, with the result that it is possible to perform effective heat exchange. In the reaction column R, the suspension of the reaction raw materials 9 is introduced, and thus a reaction on the catalyst is produced. In the discharge port, the oxidized organic substance (reforming raw material) and hydrogen are recovered as the product 10. When the pressure is set higher than the vapor pressure of water at the reaction temperature, a gas-liquid two-phase flow is produced. Since the hydrogen gas which is the product is separated as bubbles, the reaction equilibrium is shifted to the side of the product. Hence, the limitation of the reaction equilibrium which is generally disadvantageous at a low temperature is removed, and thus the reaction proceeds even at a low temperature. When the raw material of viscous liquid or slurry (solid) is supplied, with the flow layer, it is possible to efficiently perform the reaction while maintaining uniform fluidity.

[4]. Heat exchange type contact reaction device (in a case where waste heat is stored in a mixture solution of the organic substance and water which are the reaction raw materials)

A specific example of the contact reaction device according to the present invention is shown in Fig. 16.

Reference signs in the figure indicate the following meanings. 1: contact reaction device, 9: organic substance and water serving as reaction raw materials, 10: oxidized organic substance, water and hydrogen gas, 8H: honeycomb type structure formed by combining the supply pipe of organic substance and water with reaction catalyst layer. The introduction ports of the organic substance and water (the mixture which functions both as the reaction raw materials and the waste heat fluid) 9 are formed integrally.

In the case of a paper factory, a sugar factory, a biomass conversion processing step, a bitumen processing step, sludge and waste liquid processing step or the like, the low-temperature waste heat of 100°C or less may often be recovered as the suspended mixture solution of an organic substance and water. In the case of waste heat from a high-pressure system or in a case where waste heat is recovered as a mixture vapor or a mixing phase flow, higher temperature waste heat may be recovered. In such a case, as long as a catalyst reaction effectively proceeds, it is possible to directly perform the oxidation reaction (reforming reaction) of the organic substance without heat exchange.

The reactor that has the honeycomb type wall surface 8H is used, and thus it is possible to achieve both an effective flow and an increase in catalyst interfacial area while retaining the strength of the reactor. Even in this case, the catalyst reaction occurs on the wall surface 8H. The hydrogen gas of the product 10 is separated in phase from water. Hence, even in a reaction which is unlikely to occur at a low temperature in terms of the equilibrium theory, since the hydrogen gas separated in phase is removed out of the reaction system, it is possible to make the reaction proceed. In a case where the reaction raw materials 9 are vapor, a uniform reaction phase is formed. However, the pressure is set higher than the vapor pressure of water, and thus the hydrogen gas and the water phase can be separated in phase. With the same effects described above, it is possible to anticipate that the reaction is made to proceed.

[5]. Heat exchange type/wet wall type contact reaction device (in a case where waste heat is stored in a mixture solution of the organic substance and water which are the reaction raw materials)

A specific example (a variation of the above [4]) of the contact reaction device according to the present invention is

shown in Fig. 17.

Reference signs in the figure indicate the following meanings. 1: contact reaction device, 9: organic substance and water serving as reaction raw materials, 10: oxidized organic substance and water, 13: hydrogen gas, 8H: honeycomb type structure formed by combining the supply pipe of organic substance and water with reaction catalyst layer. The introduction ports of the organic substance and water (the mixture which functions both as the reaction raw materials and the waste heat fluid) 9 are formed integrally, and are provided thereabove. A discharge port of the hydrogen gas 13 other than the discharge port of the oxidized organic substance and water 10 is provided. As described above, the introduction ports of the organic substance and the water 9 formed integrally are provided in the upper portion and thus the reaction on the honeycomb type structure is performed in the method of a wet wall column. Even in this case, the catalyst reaction occurs on the honeycomb type wall surface 8H. The oxidized (reformed) organic substance and water 10 are recovered from a column bottom portion, and the hydrogen gas 13 is recovered from the upper portion. In this example, the phase separation of the generated water is effectively produced, and thus it is possible to merge the reaction step with the hydrogen separation step. Even when the product is in the form of vapor, if a pressure equal to or more than the vapor pressure is placed, it is also possible to separate the hydrogen gas. Hence, it is possible to form the reaction system without the limitation of equilibrium.

[6]. Catalyst suspension chamber type contact reaction device (in a case where waste heat is stored in a mixture solution of the organic substance and water which are the reaction raw materials)

A specific example of the contact reaction device according to the present invention is shown in Fig. 18.

Reference signs in the figure indicate the following meanings. 1: contact reaction device, 9: organic substance and water serving as reaction raw materials, 10: oxidized organic substance and water, 13: hydrogen gas, 14: agitator, S: suspended phase containing particles to which metal oxide catalyst is carried. The introduction ports of the organic substance and water (the mixture which functions both as the reaction raw materials and the waste heat fluid) 9 are formed integrally, and are provided thereabove. A discharge port of the hydrogen gas 13 other than the discharge port of the oxidized organic substance and water 10 is provided.

The suspended phase S containing the particles to which the metal oxide catalyst is carried is included as described above, and thus it is possible to obtain the oxidized organic substance and hydrogen gas such that they are separated while the particles are being retained in the suspended phase. If the catalyst suspended phase S is used, it is possible to reduce a reaction inhibition caused by the adsorption of the generated hydrogen gas to the surface of the catalyst. Furthermore, as in the example described above, even when the product is in the state of vapor, if the pressure is equal to or more than the vapor pressure is placed, it is also possible to separate the hydrogen gas. Hence, as described above, it is possible to avoid the limitation of equilibrium.

Preferably, within the contact reactor, the pressure in the reaction system is set to the saturated vapor pressure or more, and in at least part thereof, the water in the liquid phase is set to the reaction phase (the water in the liquid phase is mainly set to the reaction phase arbitrarily and selectively). In this case, the reaction temperature is set equal to or more than 300°C, and preferably equal or less than 370°C, and thus the generated hydrogen gas is separated in phase, with the result that it is possible to shift the reaction equilibrium of the elementary reaction in the oxidation of the organic substance to the side of the proceeding of the reaction. This aspect is industrially advantageous in that even when $\Delta G > 0$, it is possible to make the oxidation reaction of the organic substance proceed.

Furthermore, the reaction device according to the present invention is utilized, and thus it is possible to form the system below.

(1) A system that includes a separation chamber for separating, in the exit of a contact reactor, after cooling, a gaseous product whose main component is hydrogen gas and a product mixture solution, that further includes a recovery chamber for the gaseous product and a recovery chamber for the product mixture solution and that performs, based on the larger amount of generation and distribution of the gaseous product and the product mixture solution, pressure control for removing the product from the separation chamber to the recovery chamber.

(2) A system in which in order for the pressure of the system to be stably controlled, in the exit of the contact reactor, after cooling, a gaseous product and a liquid product are passed through a pipe whose inside diameter is equal to or less than 5 inches to constantly produce a slag flow.

In these systems, the separation chamber, the recovery chamber, the pipe and the devices for controlling the pressure are not particularly limited, and known ones can be used.

## Examples

Although the present invention will be illustrated below using examples, these examples are not limited the present invention at all.

Example of synthesis of cerium oxide in the shape of a cube

The nanoparticles of cerium oxide in the shape of a cube were synthesized by the following method.
This method is briefly described as three steps:

(i) A step of preparing a precursor (raw material) solution in toluene,
(ii) A step of using an organic modifier to synthesize, under conditions of supercritical water, the nanoparticles of $CeO_2$ in the shape of a cube and
(iii) A step of removing the organic modifier without changing the form of $CeO_2$ in the shape of a cube

In toluene (99.5%, Wako Chemical, Ltd.), as the organic modifier, hexanoic acid (99%, Wako Chemicals, Ltd., 0.30 mol / L) and Ce $(OH)_4$ (Aldrich Chemicals, Ltd., 0.050 mol / L) were dissolved, and thus the precursor solution was prepared. In order for the clear solution to be obtained, the precursor was mixed for 40 minutes while being continuously agitated. The precursor solution was supplied at a flow rate of 7.0 mL/minute with a high-pressure pump (Nihon Seimitsu Kagaku Co., Ltd., NP-KX540). At the same time, deionized water was supplied at a flow rate of 3.0 mL/minute with another pump. The precursor solution was mixed with the deionized water in a junction, and was rapidly heated to 653 K with a furnace. A residence time in a heating zone was about 95 seconds, and this was estimated from the volume of a rector, the total flow rate, the density of a mixture of water and toluene at a mixing point, the reaction temperature and the pressure. Then, the mixture was cooled with a water jacket. With a back pressure adjustment device (TESCOM, 26-1700 series), the pressure of the system was maintained to be 30 MPa. The nanoparticles of cerium oxide in the shape of a cube were obtained as a dispersion product in the mixture of water, toluene and an unreacted raw material. The sample was left for one night such that the phases of water and toluene were separated. Then, ethanol was added to the toluene phase, the mixture was purified by being subjected to three cycles of centrifugation and decanting and thus unreacted organic molecules were removed. The particles were dispersed in cyclohexane and were freeze dried under vacuum for 8 hours. The form and size of the nanoparticles were observed at an acceleration voltage of 100 kV with a transmission electron microscope (TEM, Hitachi H7650). In order to check chemical bonds and functional groups on the surface of the nanoparticles, a JASCO FT/IR-680 spectrometer was used to obtain a Fourier transform infrared spectroscopy (FTIR) spectrum. Transmission IR spectra were collected from 400 to 4000 $cm^{-1}$. The crystallinity and the purity of the particles were identified at a setup of $2\theta$ - $\theta$, by the X-ray diffraction (XRD, Rigaku Ultima IV) of Cu $K\alpha$ radiation. The angel of $2\theta$ was scanned between 20° and 70°. In order for any organic ligand to be removed from the surface of the particles, the collected nanoparticles were calcined in air for 2 hours at a temperature of 300°C within a muffle furnace whose temperature was programed at a temperature rising rate of 2°C/minute. The calcined nanoparticles were purified in ethanol a few times, and thus any unreacted molecules were removed by centrifugation and decanting. Finally, the particles were dried for 6 hours under reduced pressure, and thereafter an OSC measurement was performed on the calcined nanoparticles. In order to the OSC to be determined, the entire sample was alternately exposed to $O_2$ and He every 20 minutes.
Fig. 1 shows an XRD pattern of the nanoparticles of $CeO_2$ which were generated. In Fig. 1, (a) represents $CeO_2$ which was synthesized in the presence of hexanoic acid, (b) represents the nanoparticles of $CeO_2$ in the shape of a cube which were calcined at 300°C, (c) represents the nanoparticles of $CeO_2$ in the shape of a cube after being subjected to a reaction at 450°C, (d) represents the nanoparticles of $CeO_2$ in the shape of an octahedron which were synthesized and (e) represents the nanoparticles of $CeO_2$ in the shape of an octahedron after being subjected to a reaction at 450°C. It was found that the obtained nanoparticles have a $CeO_2$ crystal structure by comparison with the card of Joint Committee on Powder Diffraction Standards (JCPDS) obtained from International Center for Diffraction Data (00-034-0394). XRD peaks in Figs. 1a - 1c are broad as compared with peaks in Figs. 1d - 1e, and this indicates that the size of the nanoparticle shown in Figs. 1a - 1c is smaller than that of the nanoparticle shown in Figs. 1d - 1e. The sizes of crystals evaluated by a formula of Scherrer were 8nm and 50 nm on the nanoparticles shown in Fig. 1 (on each of a - c and d - e).
The size and form of the particles were analyzed with the TEM. Fig. 2 shows the form of the nanoparticles of cerium oxide which were synthesized. Figs. 2a - 2b show TEM images of the nanoparticles of cerium oxide which were synthesized at 613 K without hexanoic acid. Fig. 2c shows an image of the nanoparticles of cerium oxide which were synthesized at 653 K together with hexanoic acid. Fig. 2d shows the shape of cerium oxide calcined at 573 K. Fig. 2e shows the nanoparticles of cerium oxide in the shape of a cube after being calcined at 923 K and being used. Two types of particles: particles which were surrounded by eight {111} planes and which were in the shape of an octahedron and particles which were surrounded by six {100} planes and which were in the shape of a cube are shown in the images. The development of the particle shape from the octahedron to the cube was caused by a preferential interaction between the hexane acid ligand molecule and the {001} plane in the shape of an octahedron whose tip end was cut, thus the growth rate of the crystal in the {001} direction was significantly reduced and the crystal growth in the {111} direction was preferential and this finally led to the formation of the nanocube. It is remarkable that the size obtained based on

the XRD measurement matched with the size determined from the TEM analysis.

In order to prove the presence of organic molecules chemically bound to both surfaces of the nanoparticles of $CeO_2$ in the shape of a cube, an FTIR spectrum was obtained. As shown in Fig. 3(a), in the surface-modified nanoparticles, in a region of 2900 - 2970 $cm^{-1}$, expansion/contraction peaks appeared. These peaks were allocated to the C-H expansion/contraction mode of a methyl group and a methylene group in hexane acid, were present in the FTIR spectrum of the net modifier and indicate the presence of organic molecules on the surface of the nanoparticles. In the spectrum (Fig. 3(a)) of the nanoparticles modified by hexane acid, two main peaks in 1531 and 1444 $cm^{-1}$ were respectively allocated to the asymmetric and symmetric modes of a carboxylate group. This indicates that hexane acid is chemically bound by the carboxylate group to the surface of the nanoparticles of cerium oxide. It was necessary to remove the organic ligand bound to the surface of the catalyst before the nanoparticles of cerium oxide were used as the reaction catalyst, without any change of the form of the particles. This is because the organic ligand bound to the surface of the catalyst can function as the reactant. Furthermore, the organic ligand bound to the surface of the catalyst can inhibit an interaction between the reaction substance and the surface of the catalyst. Hence, organic molecules need to be removed from the surface of the catalyst. The thermal processing was selected as a general method for removing the organic ligand from the surface of the particles. Organic molecules are easily decomposed into $CO_2$ and $H_2O$ during combustion in a current of air. The FTIR spectrum of the particles calcined at 300°C in Fig. 3(b) indicates that the nanoparticles were calcined and then the presence of the organic molecules was reduced. Thereafter, the form of the particles was examined by TEM analysis, and thus it was confirmed that no variation was produced during the calcination (Fig. 2d). However, in order for coke formed on the catalyst to be removed, the nanoparticles of $CeO_2$ which were used were calcined at 923 K. It indicates that a small variation was produced during the calcination (Fig. 2e). It means that since the reaction temperature (723 K) is lower than the calcination temperature, the shape of the nanoparticles of $CeO_2$ is stabilized at the reaction temperature.

OSC evaluation of the nanoparticles of $CeO_2$

The oxygen storage capacity (OSC) is defined as the amount of oxygen which is stored in the catalyst and is discharged therefrom. In order to determine the OSC whose total was utilized and evaluate the potential activity thereof for the contact reaction, the OSC of the nanoparticles of $CeO_2$ in the shape of a cube and an octahedron was measured at 723 K according to the above method example 1. The results thereof indicated that the OSC of the nanoparticles of cerium oxide in the shape of a cube was 340 $\mu$mol - $O_2g^{-1}$ which was about 3.4 times higher than the OSC (100 $\mu$mol - $O_2g^{-1}$) of the nanoparticles of cerium oxide in the shape of an octahedron at 723 K. The nanoparticles of cerium oxide in the shape of a cube whose size and activity were lower and which included a {100} plane had a higher OSC. These results are caused by a larger exposure surface area in the smaller nanoparticles, and indicate that oxygen molecules involved in the oxygen storage/discharge process are mainly located on the surface of $CeO_2$.

OSC evaluation of metal oxide nanoparticles which are various solid electrolytes

With respect to metal oxide nanoparticles which are $CeO_2$ - 100, $CeO_2$ - 111 and other solid electrolytes, in order to examine the availability thereof as the catalyst of the present invention, the OSC of the following metal oxides at 350°C was measured according to the above method example 2. The measurement targets here are $CeO_2$ - 100, $CeO_2$ - 111, LSM (lanthanum strontium manganite), Gd - $CeO_2$ (gadolinium-doped cerium oxide), $In_2O_3$ (indium oxide), $MoO_3$ (molybdenum oxide), YSZ (yttrium stabilized zirconium oxide) and LSCF (lanthanum strontium cobalt ferrite). The measurement results of the OSC on the metal oxides including $CeO_2$ - 100 and $CeO_2$ - 111 are shown as a bar graph in Fig. 19.

It has been found from the measurement results that any of the metal oxides indicated here has a significant OSC. Specifically, the lowest OSC among them was 50 $\mu$mol - $O_2g^{-1}$ for YSZ. Hence, it can be considered that any of the metal oxides of these solid electrolytes is available as the catalyst in the processing method of the present invention.

Evaluation of temperature dependence of OSC of metal oxide nanoparticles

The OSC of a plurality of types of metal oxide nanoparticles (Gd - $CeO_2$ (gadolinium-doped cerium oxide) and YSZ (yttrium stabilized zirconium oxide) when the temperature was changed to 35°C, 100°C, 200°C and 350°C was measured according to the above method example 2, and the temperature dependence of the OSC was examined. Variations in the OSC of each of the metal oxide nanoparticles are shown in Fig. 20 together with the OSC of $CeO_2$ - 100 ($CeO_2$ in the shape of a cube) at 350°C, $CeO_2$ - 111 ($CeO_2$ in the shape of an octahedron) and $In_2O_3$ (indium oxide).

As expected, it has been found that the OSC of each of the metal oxide nanoparticles is gradually increased with an increase in temperature. In other words, it has been found that the OSC of each of the metal oxide nanoparticles is gradually decreased with a decrease in temperature. The OSC of any of the metal oxides had a significant value at 35°C.

In examples 1 to 3 below, $CeO_2$ - 100 ($CeO_2$ in the shape of a cube) synthesized as described above was used as the metal oxide catalyst of the solid electrolyte.

Example 1: Oxidative decomposition of acetaldehyde

Since in various synthesis reactions, carboxylic acid is an important intermediate, the oxidation of aldehyde to the corresponding carboxylic acid is considered as one of the important methods in organic synthesis. The Cannizzaro reaction which proceeds under no catalyst is a redox reaction in which a hydroxide base is used to make two molecules of aldehyde react to generate a primary alcohol and carboxylic acid. A great deal of research has been performed on the oxidation of aldehyde using molecular oxygen as an oxidizing agent. Examples thereof include an aerobic homogeneous catalyst system such as [Ni $(acac)_2$] and an aerobic heterogeneous catalyst system such as Au/C and Ru/$CeO_2$.

Here, in order to evaluate the performance of the $CeO_2$ catalyst, another test was performed. The cerium oxide in the shape of a cube synthesized as described above (20 mg) and 2.57 ml of an acetaldehyde aqueous solution (2.0 M) were put into a reactor. The reaction temperature was set at 350°C. For comparison, the reaction was also performed under no catalyst.

The concentration of the product was examined using a peak area in a GC-MS spectrum and DMS as an internal standard.

A result when the catalyst ($CeO_2$) was not used is first indicated. It has been found from a chromatograph (Fig. 4 - a) that the main products were acetic acid, ethanol, ethyl acetate and an aldol condensation product. It has been found from diagrams (Fig. 5 - a and Fig. 5 - b) where the yield is plotted with respect to the time that as acetaldehyde is reduced, acetic acid and ethanol are increased. Since the generation of ethyl acetate is caused by the dehydration (esterification) of acetic acid and ethanol, when it is considered that a half of the yield is acetic acid and ethanol, it can be understood that acetic acid and ethanol are the main products. These products are products that are generated by the Cannizzaro reaction ($CH_3CHO + CH_3CHO = CH_3COOH + CH_3OH$). The aldol condensation product is generated by $CH_3CHO + CH_3CHO = CH_3C(OH)CH_3CHO$. It has been found by comparison of the yields that about the same amounts of product of the Cannizzaro reaction and product of the aldol condensation reaction were produced.

A result when the catalyst ($CeO_2$) was used is then indicated. It can be understood that although the same products as when the catalyst was not used were produced, a larger amount of aldol condensation product was produced, and the methoxylation of acetate proceeds. The methoxylation suggests the generation of methanol and indicates the possibility that acetic acid was further oxidized and was decomposed into methanol and $CO_2$. The generation of gases was confirmed, and a few percent of methane and $CO_2$ was generated. It can be considered that methane was thermally decomposed simultaneously when acetaldehyde was oxidatively decomposed and was generated simultaneously when $CO_2$ was generated. This is a valid result in terms of the oxidation of acetic acid and a slightly higher yield of $CO_2$.

It has been found from the diagrams (Fig. 5 - a and Fig. 5 - b) where the yield is plotted that the yield of acetaldehyde is more reduced and the reaction is facilitated by the presence of the catalyst $CeO_2$. The yield of ethanol is substantially equal thereto. However, when the amount of ethanol generated is compared with consideration given to the fact that ethyl acetate is generated from ethanol and acetic acid (a half of the yield thereof is added), it can be understood that the amount of ethanol generated when $CeO_2$ is added is lower.

When as in the above discussion, it is considered that the oxidation reaction of the catalyst $CeO_2$ occurs in ethanol, it can be understood that the estimated amount of ethanol generated when $CeO_2$ is added is lower. Likewise, it is possible to provide an explanation for the larger amount of acetic acid generated and the enhanced reaction rate of acetaldehyde. Since a larger amount of acetic acid generated facilitates the esterification reaction with the aldol condensation product, it can be understood that the amounts of these products are increased. It can be considered that a smaller amount of ethanol generated leads to the reduction of the generation of ethyl acetate.

It has been confirmed from these results that reactions represented by the following formulas are produced.

$$CH_3CHO + (O) = CH_3COOH \ (\Delta G^0_f = -421.9 \text{ kJ/mol})$$

$$CH_3CHO + (O) = CH_4 + CO_2 \ (\Delta G^0_f = -220.03 \text{ kJ/mol})$$

$$CH_3COOH + (O) = CH_3OH + CO_2 \ (\Delta G^0_f = -36.63 \text{ kJ/mol})$$

$$CH_3CH_2OH + (O) = CH_3CHO \ (\Delta G^0_f = -343.01 \text{ kJ/mol})$$

where $\Delta G^0_f$ represents a variation in Gibbs free energy in a standard state calculated by using the HKF model.

With respect to the reaction in which acetic acid ($CH_3COOH$) is generated from acetaldehyde ($CH_3CHO$), the balance of the theoretical calculation of a variation in Gibbs free energy including the oxidation and reduction of the catalyst $CeO_2$ is as follows.

1)

$CH_3CHO + 2CeO_2 \rightarrow CH_3COOH + Ce_2O_3$

2)

$H_2O + Ce_2O_3 \rightarrow 2CeO_2 + H_2$

Sum) $CH_3CHO + H_2O \rightarrow CH_3COOH + H_2$

1)

$$\Delta G = (-1706.2 - 389.9) - (-2*1024.6 - 128.2) = 81.3 \text{ kJ/mol } (\Delta H° = 89.1 \text{ kJ/mol})$$

2)

$$\Delta G = (2*-1024.6 + 0) - (-228.61 - 1706.2) = -114.39 \text{ kJ/mol}$$

$$\text{Total) } \Delta G = (-389.9 + 0) - (-128.2 - 228.61) = -33.1 \text{ kJ/mol}$$

Example 2: Oxidative decomposition of lignin

In the decomposition reaction of lignin (copolymer of a guaiacol skeleton and glyceraldehyde) under hydrothermal conditions, not only hydrolysis but also the retro aldol reaction of aldehyde is produced, and a larger amount of aldehyde is generated. The aldehyde is subjected to Friedel-Crafts reaction with a phenol skeleton to advance the polymerization. Hence, although the decomposition proceeds, the polymerization also proceeds. If the aldehyde can be converted into a carboxylic acid by being oxidized, since a carboxylic acid does not react with phenol in terms of the Friedel-Crafts reaction, it is possible to reduce the polymerization.

Hence, under hydrothermal conditions shown in Fig. 6, the catalyst $CeO_2$ was used in the proportion of 3 or 10 to the weight of lignin, and thus the oxidative decomposition of lignin was performed. A batch type reactor was used, and thus the reaction was performed at 300 to 400°C for 10 minutes, and thereafter rapid cooling was performed.

As shown in Figs. 7 to 9, in any of the cases using 400°C / 300 mg of the catalyst (supercritical water), 350°C / 1000 mg of the catalyst and 300°C / 1000 mg of the catalyst, it was visually recognized that the oxidative decomposition of lignin proceeded (the reaction solution was more purified). It was confirmed that as the amount of catalyst was increased, the decomposition was clearly facilitated. In the case of 1000 mg of the catalyst, it was confirmed that even at a low temperature of not only 350°C but also 300°C, the decomposition reaction was produced.

Example 3: Oxidative decomposition of pigment

In order to check whether or not the oxidative decomposition reaction is produced even at a lower temperature, a test was performed using, as a model molecule, a pigment molecule which is more easily visually recognized.

As the pigment, indigo carmine (acid blue 74) was used which is commonly known as indigo chin represented by the following formula. This compound acts as a pH indicator and a redox indicating agent in a chemical reaction.

[Chemical Formula 1]

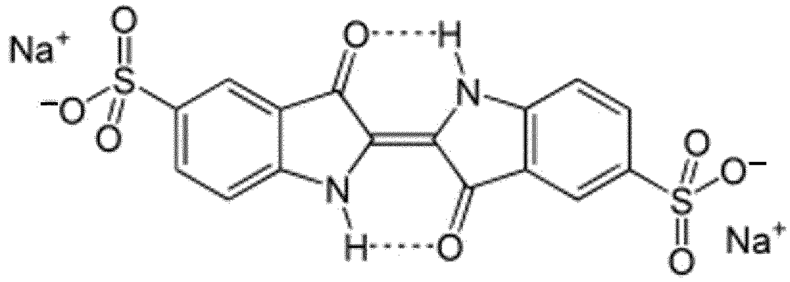

Cerium oxide in the shape of a cube (20 mg) and 2.0 ml of an aqueous solution of the pigment (0.01 M) were put into a reactor. The reaction temperature was set at 250 to 350°C. Fig. 10 (without use of the catalyst) shows that the pigment was not changed after the reaction. This means that without the presence of the catalyst, a large proportion of the pigment was not decomposed under hydrothermal conditions before the criticality. However, as shown in Fig. 11, when under the same temperature conditions, the nanocatalyst of $CeO_2$ in the shape of a cube was used, the decomposition was facilitated. Surprisingly, it was confirmed that even at a low temperature of 250°C, the decomposition reaction was remarkably facilitated.

In examples 4 to 6 below, tests were performed using a metal oxide catalyst which is a solid electrolyte other than $CeO_2$ - 100 ($CeO_2$ in the shape of a cube).

Example 4: Oxidative decomposition of pigment (in the presence of a metal oxide catalyst other than cerium oxide in the shape of a cube

Each catalyst (100 mg) and 2.5 ml of an aqueous solution of pigment/indigo carmine (0.1 mM) were put into a reactor. The reaction temperature was set at 100°C, and the reaction was performed for 1 hour. The catalysts used were Molybdenum oxide ($MoO_3$), manganese oxide ($MnO_3$), indium oxide ($In_2O_3$), iron oxide ($Fe_2O_3$), tetroxide three cobalt ($Co_3O_4$), lanthanum strontium cobalt ferrite (LSCF), lanthanum strontium ferrite (LSF), cobalt oxide II (CoO) and vanadium oxide ($V_2O_5$). For comparison, the reaction was likewise performed on a pigment aqueous solution excluding the catalyst. The appearances of the solutions after the oxidation reaction was performed in the present of various types of metal oxide catalysts are shown in Fig. 21 together with reference water.

By using UV-VIS (ultraviolet-visible spectroscopy), a decrease in absorbance before and after the reaction was measured at a peak of 611 nm, and thus the decomposition rate of the raw material was quantified. The values of the raw material decomposition rate are also shown in Fig. 21. The experimental error of the values is expected to be about $\pm$ 0.5%. It can be considered that even in the case of no catalyst, a slight reaction occurred due to an influence from the surface of the reactor. In the case of $MnO_3$, the raw material decomposition rate was substantially equal to that in the case of no catalyst. It has been found that in a case where metal oxide catalysts other than it were used, the reaction proceeded significantly in any of the metal oxide catalysts.

Example 5: Oxidative decomposition of acetaldehyde (in the presence of a metal oxide catalyst other than cerium oxide in the shape of a cube)

A metal oxide catalyst (100 mg) of a solid electrolyte and 2.5 ml of an acetaldehyde aqueous solution (1.0 M) were put into a reactor. The reaction temperature was set at 350°C, and the reaction time was set to 30 minutes.

As for the metal oxide catalyst, any one of Yttrium-stabilized zirconium oxide (YSZ), lanthanum strontium manganite (LSM), gadolinium-doped cerium oxide (Gd-CeO2), scandium oxide ($Sc_2O_3$), lanthanum oxide gallium ($LaGaO_3$), iron oxide ($Fe_2O_3$), indium oxide ($In_2O_3$) and scandium oxide-doped zirconium oxide (ScZ: also referred to as ScSZ) was used.

The yields of acetaldehyde (residue) and acetic acid calculated in these reactions based on the carbon molar concentration standard of the whole of acetaldehyde of the raw material are shown in Fig. 22. Even when any of the catalysts was used, the reaction proceeded with significantly high yield, with the result that acetic acid which was an oxidation product was obtained.

Example 6: Oxidative decomposition of propanal (in the presence of a metal oxide catalyst other than cerium oxide in the shape of a cube)

A metal oxide catalyst (100 mg) of a solid electrolyte and 2.5 ml of a propanal aqueous solution (1.0 M) were

put into a reactor. The reaction temperature was set at 350°C, and the reaction time was set to 30 minutes.

As for the metal oxide catalyst, any one of cerium oxide in the shape of a cube, vanadium oxide ($V_2O_5$) and cobalt oxide II (CoO) synthesized as described above was used. For comparison, the reaction was also performed under no catalyst.

The yields of propanal (residue) and propionic acid (oxidation product) calculated in these reactions based on the carbon molar concentration standard of the whole of propanal of the raw material were as follows. Although it can be understood that the reaction was complicated, even when any of the catalysts was used, the reaction proceeded with significantly high yield, with the result that propionic acid which was an oxidation product was obtained.

[Yield of propanal (residue)]

- No catalyst: 77.7%
- Cerium oxide ($CeO_2$) in the shape of a cube: 43.5%
- Vanadium oxide ($V_2O_5$): 31.0%
- Cobalt oxide II (CoO): 41.3%

[Yield of propionic acid]

- No catalyst: 1.7%
- Cerium oxide ($CeO_2$) in the shape of a cube: 1.7%
- Vanadium oxide ($V_2O_5$): 1.9%
- Cobalt oxide II (CoO): 1.9%

Reference Signs List

1: contact reaction device, 2: contact reactor, 3: region including reaction catalyst layer containing metal oxide catalyst, 4: introduction port of organic substance, 5: introduction port of water, 6: discharge port of oxidized organic substance, 7: discharge port of hydrogen gas, 8: reaction catalyst layer, 8H: honeycomb type structure formed by combining supply pipe of organic substance and water with reaction catalyst layer, 9: organic substance and water serving as reaction raw materials, 10: oxidized organic substance and water (oxidized organic substance, water and hydrogen gas), 11: waste heat fluid, 12: fluid of waste heat recovery, 13: hydrogen gas, 14: agitator, R: reaction column, C: catalyst flow column, m: particles to which metal oxide catalyst is carried, S: suspended phase containing particles to which metal oxide catalyst is carried

## Claims

**1.** A method of processing an organic substance under a hydrothermal condition by utilizing an oxidation-reduction cycle of a metal oxide catalyst, is the method comprising:

(i) oxidizing an organic substance with oxygen discharged from a metal oxide catalyst having an oxidized metal value so as to form a metal oxide catalyst having a reduced metal value and an oxidized organic substance; and
(ii) oxidizing, simultaneously with the above (i), the metal oxide catalyst having the reduced metal value with oxygen discharged from water so as to reproduce the metal oxide catalyst having the oxidized metal value,

wherein the metal oxide catalyst is a solid electrolyte.

**2.** The method according to claim 1,
wherein in the above (i) and (ii) as a whole, $\Delta H$ (under the hydrothermal condition) > 0, and
a reaction product obtained from the organic substance and water contains an oxidized organic substance and a hydrogen gas.

3. The method according to claim 2, wherein the reaction product contains, in addition to the oxidized organic substance and the hydrogen gas, a substance obtained by further hydrogenating the oxidized organic substance.

4. The method according to claim 1, wherein a reaction product obtained from an organic substance and water contains a substance obtained by further hydrogenating an oxidized organic substance.

5. The method according to any one of claims 1 to 4, wherein in the presence of supercritical water or water before criticality, reactions of the above (i) and (ii) are performed.

6. The method according to any one of claims 1 to 4, wherein the metal oxide catalyst is selected from a group consisting of cerium oxide ($CeO_2$), indium oxide ($In_2O_3$), iron oxide ($Fe_2O_3$), yttrium-stabilized zirconium oxide (YSZ), scandium oxide-doped zirconium oxide (ScSZ), scandium oxide ($Sc_2O_3$), oxidation lanthanum gallium ($LaGaO_3$), lanthanum strontium manganite (LSM), gadolinium-doped cerium oxide (Gd-$CeO_2$), molybdenum oxide ($MoO_3$), manganese oxide ($MnO_3$), lanthanum strontium cobalt ferrite (LSCF), lanthanum strontium ferrite (LSF), tetroxide three cobalt ($Co_3O_4$), cobalt oxide II (CoO), vanadium oxide ($V_2O_5$) and ceria-zirconia solid solution.

7. The method according to claim 6, wherein the metal oxide catalyst contains a nanoparticle of the cerium oxide ($CeO_2$).

8. The method according to claim 7, wherein the metal oxide catalyst is formed in a shape of an octahedron and/or a cube, and contains the nanoparticle of the cerium oxide ($CeO_2$) in which a (111) plane and/or a (100) plane is a main exposure plane.

9. The method according to any one of claims 1 to 8, wherein a large proportion of the organic substance is formed of a hydrocarbon.

10. The method according to claim 9, wherein the above oxidation (i) includes oxidative decomposition of a hydrocarbon.

11. The method according to any one of claims 1 to 8, wherein the organic substance contains a substance that is selected from an aldehyde, sludge, lignin, plastic waste and biomass waste.

12. The method according to any one of claims 1 to 11, wherein the reactions of the above (i) and (ii) are performed at a temperature that is equal to or more than a room temperature but less than 450°C.

13. The method according to any one of claims 1 to 12, wherein the above oxidation (i) includes the oxidative decomposition of the hydrocarbon, and an oxidatively decomposed organic substance is further hydrogenated with hydrogen derived from water into a product that has a lower molecular weight.

14. The method according to claim 13, wherein after hydrogenation, a ratio of a molar yield of a saturated hydrocarbon to a molar yield of an unsaturated hydrocarbon is higher than in a decomposition reaction under no catalyst.

15. The method according to any one of claims 1 to 3 and 5 to 14, wherein a reaction temperature is set equal to or less than 370°C, a pressure within a reaction system is set equal to or more than a saturated vapor pressure, at least a part of water is in a liquid phase and generated hydrogen gas is separated in phase such that a reaction equilibrium of the above oxidation (i) is shifted to a reaction proceeding side.

16. A contact reaction device for processing an organic substance under a hydrothermal condition by utilizing an oxidation-reduction cycle of a metal oxide catalyst, the contact reaction device comprising:

an introduction port of each of an organic substance and water that are reaction raw materials;
a contact reactor having a reaction catalyst layer containing a metal oxide catalyst; and
a discharge port of an oxidized organic substance that is a reaction product,
wherein in the contact reactor, a reaction is performed that includes:

(i) oxidizing an organic substance with oxygen discharged from a metal oxide catalyst having an oxidized metal value so as to form a metal oxide catalyst having a reduced metal value and an oxidized organic substance; and
(ii) oxidizing, simultaneously with the above (i), the metal oxide catalyst having the reduced metal value with oxygen discharged from water so as to reproduce the metal oxide catalyst having the oxidized metal value, and

the metal oxide catalyst is a solid electrolyte.

**17.** The device according to claim 16,
wherein in the above (i) and (ii) as a whole, ΔH (under the hydrothermal condition) > 0, and a reaction product obtained from the organic substance and water contains an oxidized organic substance and a hydrogen gas, and in the contact reaction device,

the introduction ports of the organic substance and water that are the reaction raw materials are integrally formed, and
the reaction catalyst layer is provided along a wall surface of a heat exchange heat transfer pipe, a waste heat fluid is passed through the heat exchange heat transfer pipe, the waste heat fluid and the reaction raw materials are brought into contact to supply heat necessary for an endothermic reaction of the organic substance and waste heat is simultaneously recovered.

**18.** The device according to claim 17, further comprising:

a discharge port of the hydrogen gas,
wherein the heat exchange heat transfer pipe and the reaction catalyst layer are provided so as to form a predetermined angle with respect to a horizontal direction and the introduction ports of the organic substance and water formed integrally are provided thereabove such that the reaction on the reaction catalyst layer is performed in a method of a wet wall column.

**19.** The device according to claim 16,
wherein in the above (i) and (ii) as a whole, ΔH (under the hydrothermal condition) > 0, and a reaction product obtained from the organic substance and water contains an oxidized organic substance and a hydrogen gas, and in the contact reaction device,

a reaction column and a catalyst flow column are included,
in the catalyst flow column, a particle to which the metal oxide catalyst is carried absorbs heat from a waste heat fluid to recover heat and
in the reaction column, the reaction raw materials and the particle absorbing heat are brought into contact to supply heat necessary for an endothermic reaction of the organic substance.

**20.** The device according to claim 16,
wherein in the above (i) and (ii) as a whole, ΔH (under the hydrothermal condition) > 0, and a reaction product obtained from the organic substance and water contains an oxidized organic substance and a hydrogen gas, and in the contact reaction device,

the introduction ports of the organic substance and water are integrally formed such that the organic substance and water are supplied as a mixture which functions both as the reaction raw materials and waste heat fluid, and supply pipes of the organic substance and water are combined with the reaction catalyst layer to form a honeycomb type structure such that an endothermic reaction of the organic substance is performed on a wall surface of the honeycomb type structure.

**21.** The device according to claim 20, further comprising:

a discharge port of the hydrogen gas,
wherein the introduction ports of the organic substance and water formed integrally are provided thereabove such that the reaction on the honeycomb type structure is performed in a method of a wet wall column.

**22.** The device according to claim 16,
wherein in the above (i) and (ii) as a whole, ΔH (under the hydrothermal condition) > 0, and a reaction product obtained from the organic substance and water contains an oxidized organic substance and a hydrogen gas, and in the contact reaction device,

the introduction ports of the organic substance and water are integrally formed such that the organic substance and water are supplied as a mixture which functions both as the reaction raw materials and a waste heat fluid, a discharge port of the hydrogen gas is further included and
the reaction catalyst layer is formed as a suspended phase that contains a particle to which the metal oxide catalyst is carried such that while the particle is being held in the suspended phase, the oxidized organic substance and the hydrogen gas are obtained so as to be separated.

**23.** The device according to claim 16,
wherein in the above (i) and (ii) as a whole, ΔH (under the hydrothermal condition) > 0, and a reaction product obtained from the organic substance and water contains an oxidized organic substance and a hydrogen gas, and in the contact reaction device,

a heat exchange heat transfer pipe in which a waste heat fluid and reaction raw materials are brought into contact to supply heat necessary for an endothermic reaction of the organic substance and in which waste heat is simultaneously recovered is arranged so as to make contact with the reaction catalyst layer, and
another heat exchange heat transfer pipe in which after the heat exchange between the waste heat fluid and the reaction raw materials within the contact reactor, the pre-heated reaction raw materials are supplied is included.

**24.** The method according to claim 16,
wherein within the contact reactor, a reaction temperature is set equal to or less than 370°C, a pressure within a reaction system is set equal to or more than a saturated vapor pressure, at least a part of water is in a liquid phase and a generated hydrogen gas is separated in phase such that reaction equilibrium of the above (i) is shifted to a reaction proceeding side.

**25.** A system comprising the device according to any one of claims 16, 17, 19, 20 and 23, the system further comprising:

a separation chamber for separating, in an exit of the contact reactor, after cooling, a gaseous product whose main component is hydrogen gas and a product mixture solution;
a recovery chamber for the gaseous product; and
a recovery chamber for the product mixture solution,
wherein based on a larger one of an amount of the gaseous product generated and passed and an amount of the product mixture solution generated and passed, pressure control for removing the product from the separation chamber to the recovery chamber is performed.

**26.** A system comprising the device according to any one of claims 16, 17, 19, 20 and 23,
wherein in an exit of the contact reactor, after cooling, a gaseous product and a liquid product are passed through a pipe whose inside diameter is equal to or less than 5 inches (12.7 centimeters) to constantly produce a slag flow such that a pressure of the system is stably controlled.

**27.** A method of processing an organic substance under a hydrothermal condition by utilizing an oxidation-reduction cycle of a metal oxide catalyst so as to chemically recover waste heat from a low-temperature heat source, the method comprising:

(i) oxidizing an organic substance with oxygen discharged from a metal oxide catalyst having an oxidized metal value so as to form a metal oxide catalyst having a reduced metal value and an oxidized organic substance; and
(ii) oxidizing, simultaneously with the above (i), the metal oxide catalyst having the reduced metal value with oxygen discharged from water so as to reproduce the metal oxide catalyst having the oxidized metal value,

wherein the metal oxide catalyst is a solid electrolyte, and
through the oxidation-reduction cycle of the metal oxide catalyst, the waste heat is recovered as binding energy of the product which is the oxidized organic substance.

**28.** The method according to claim 27,
wherein in the above (i) and (ii) as a whole, $\Delta H$ (under the hydrothermal condition) > 0, and
a reaction product obtained from the organic substance and water contains an oxidized organic substance and a hydrogen gas.

**29.** The method according to claim 28,
wherein the reaction product contains, in addition to the oxidized organic substance and the hydrogen gas, a substance obtained by further hydrogenating the oxidized organic substance.

**30.** The method according to claim 27,
wherein the reaction product obtained from the organic substance and water contains a substance obtained by further hydrogenating the oxidized organic substance.

Fig.1

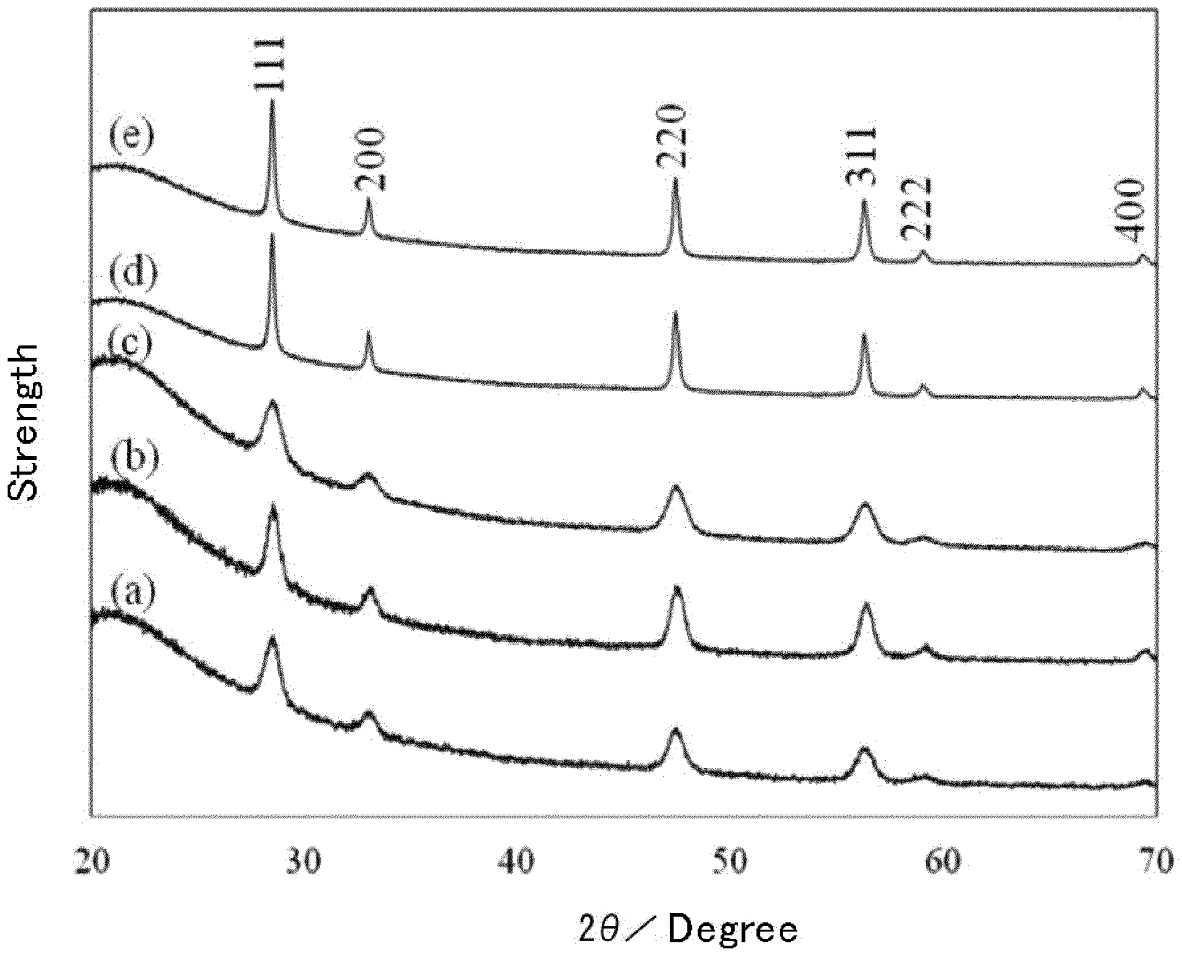
111
200
220
311
222
400
(e)
(d)
(c)
(b)
(a)
Strength
20
30
40
50
60
70
2θ／Degree

Fig.2

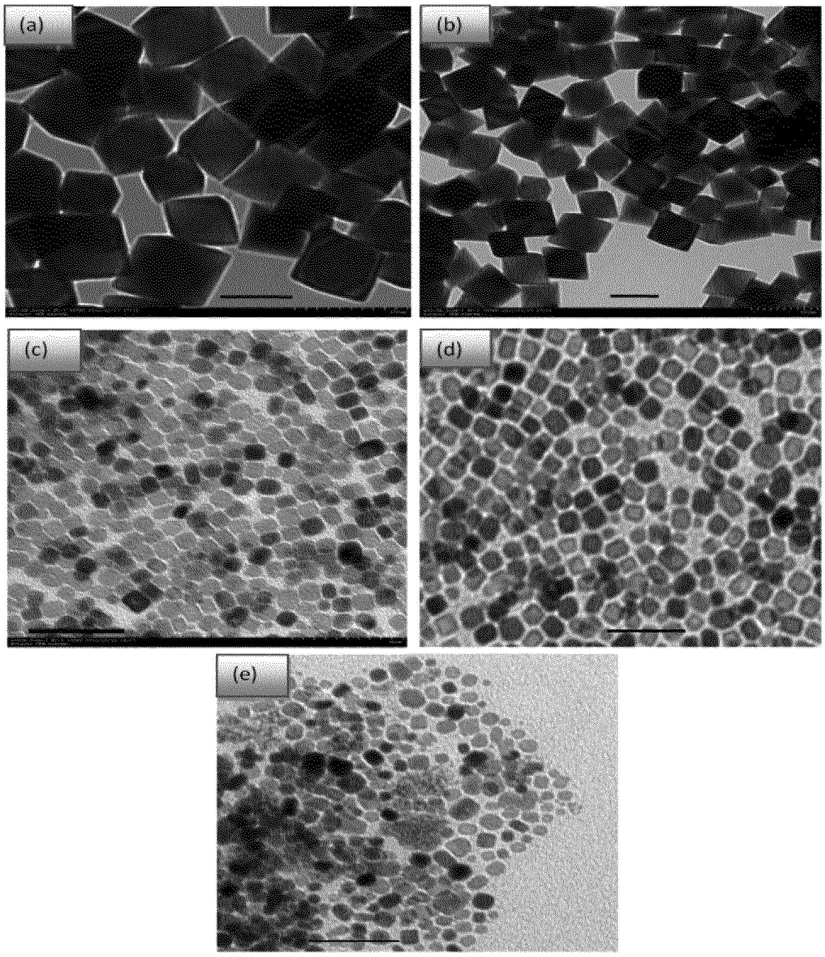
(a)
(b)
(c)
(d)
(e)

Fig.3

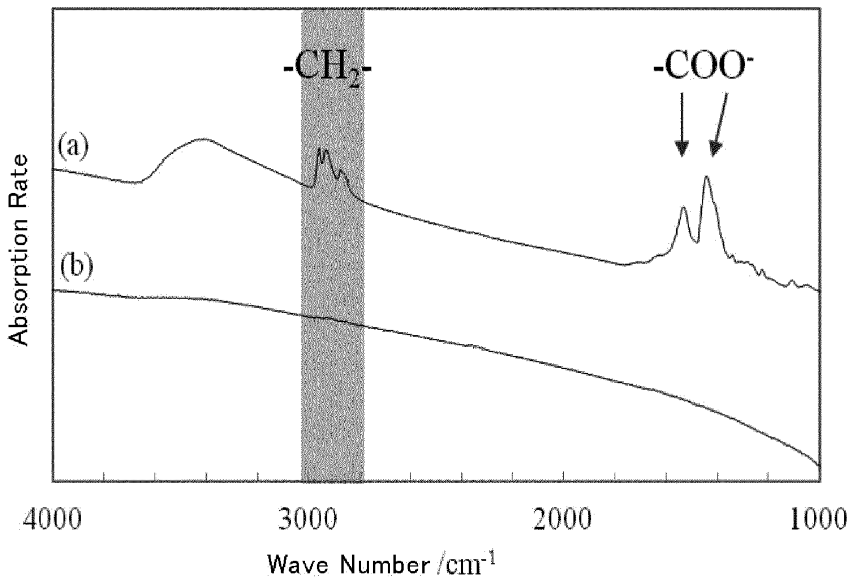
-CH2-
-COO-
(a)
(b)
Absorption Rate
4000
3000
2000
1000
Wave Number /cm-1

Fig.4-a

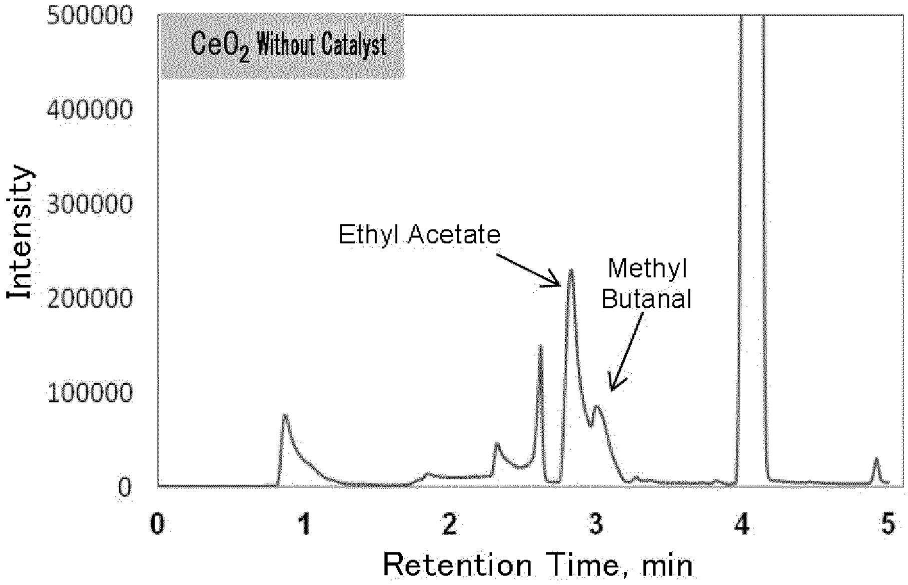
$CeO_2$ Without Catalyst
500000
400000
300000
200000
100000
0
Intensity
Ethyl Acetate
Methyl Butanal
0
1
2
3
4
5
Retention Time, min

Fig.4-b

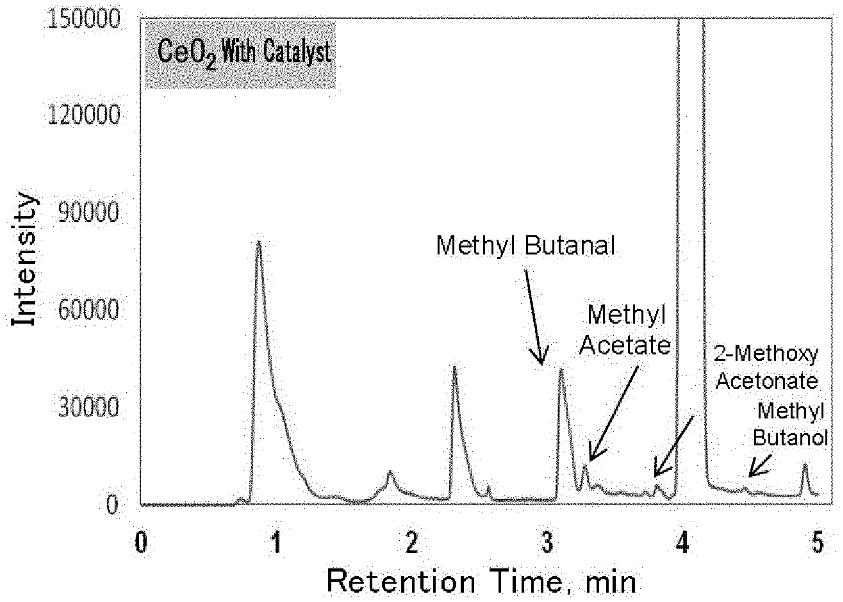
$CeO_2$ With Catalyst
150000
120000
90000
60000
30000
0
Intensity
Methyl Butanal
Methyl Acetate
2-Methoxy Acetonate
Methyl Butanol
0
1
2
3
4
5
Retention Time, min

Fig.5-a

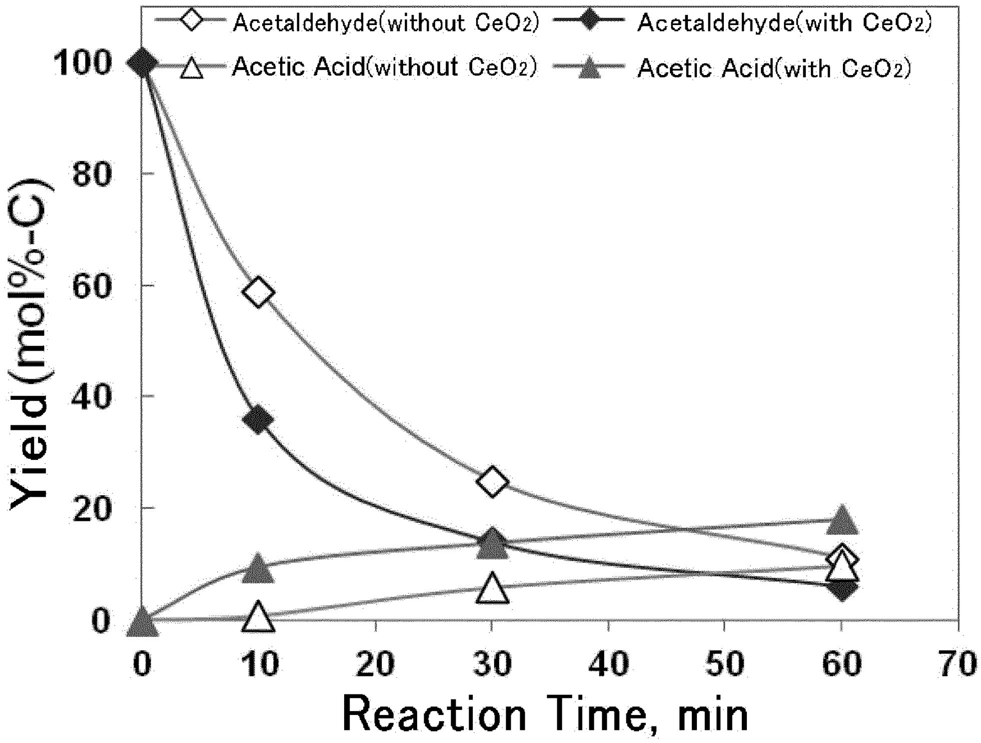
Acetaldehyde(without $CeO_2$)
Acetaldehyde(with $CeO_2$)
Acetic Acid(without $CeO_2$)
Acetic Acid(with $CeO_2$)
Yield (mol%-C)
Reaction Time, min
0
20
40
60
80
100
0
10
20
30
40
50
60
70

Fig.5-b

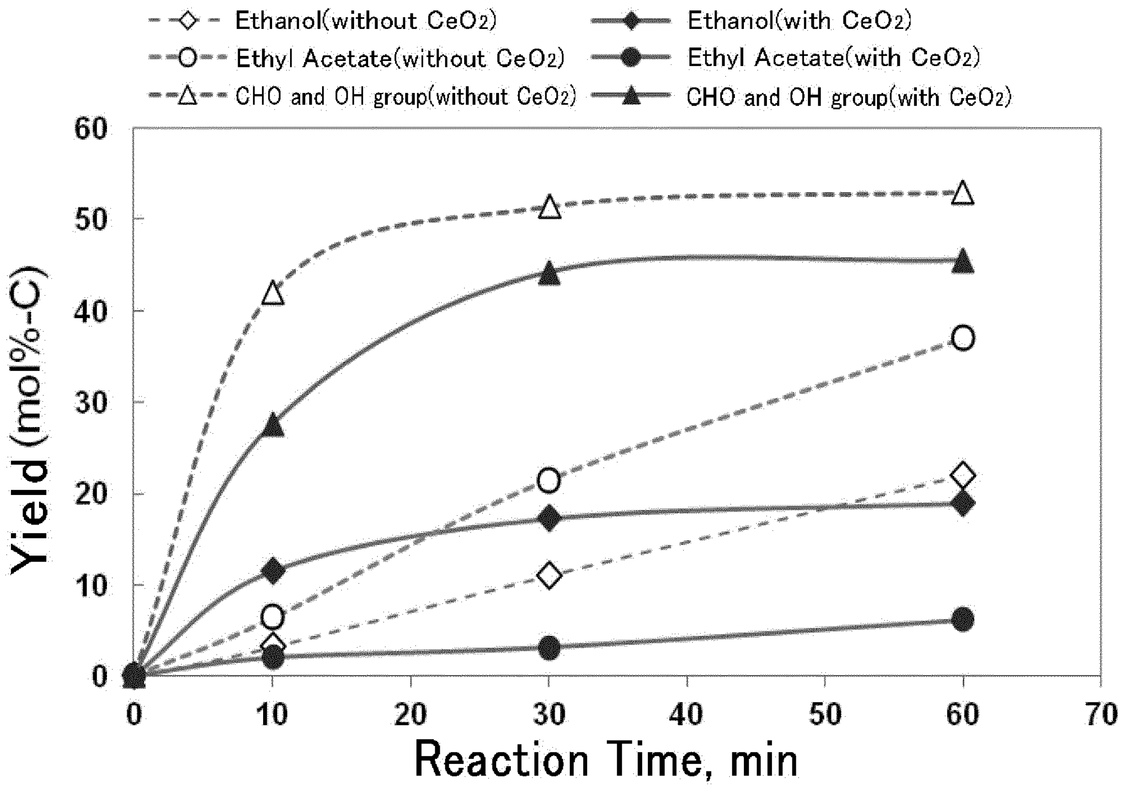
Ethanol(without $CeO_2$)
Ethanol(with $CeO_2$)
Ethyl Acetate(without $CeO_2$)
Ethyl Acetate(with $CeO_2$)
CHO and OH group(without $CeO_2$)
CHO and OH group(with $CeO_2$)
Yield (mol%-C)
Reaction Time, min
0
10
20
30
40
50
60
0
10
20
30
40
50
60
70

Fig.6

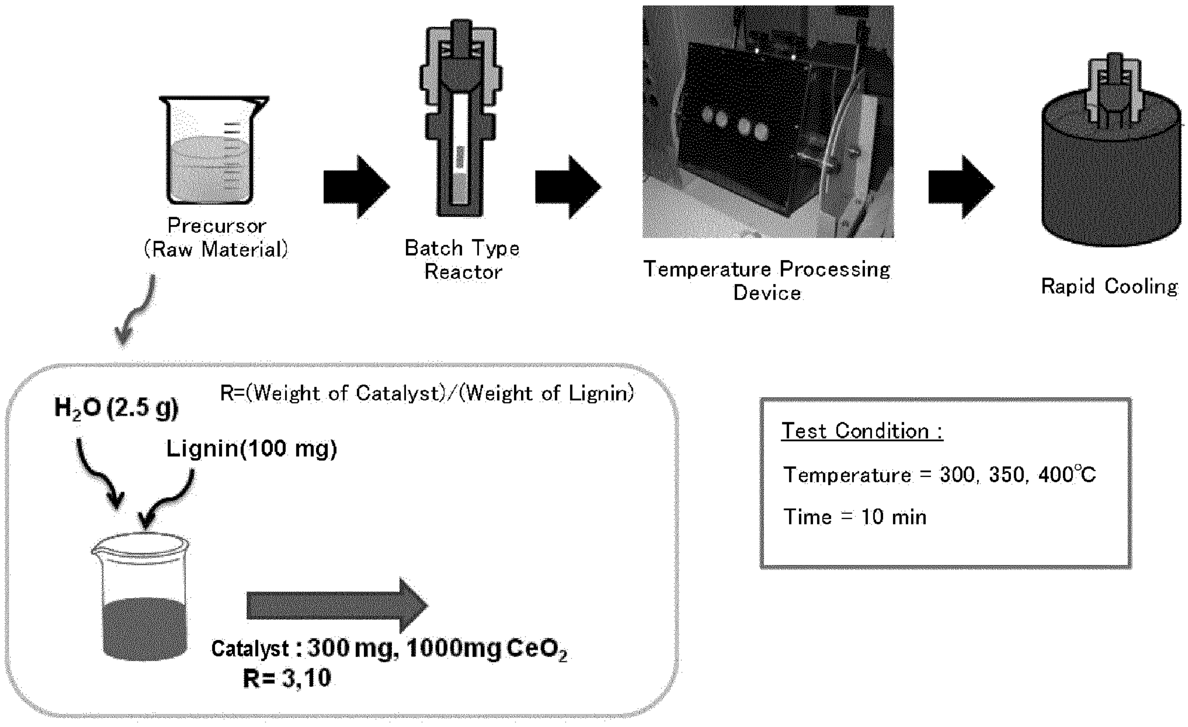
Precursor
(Raw Material)
Batch Type
Reactor
Temperature Processing
Device
Rapid Cooling
$H_2O$ (2.5 g)
R=(Weight of Catalyst)/(Weight of Lignin)
Lignin(100 mg)
Catalyst : 300 mg, 1000mg $CeO_2$
R= 3,10
Test Condition :
Temperature = 300, 350, 400°C
Time = 10 min

Fig.7

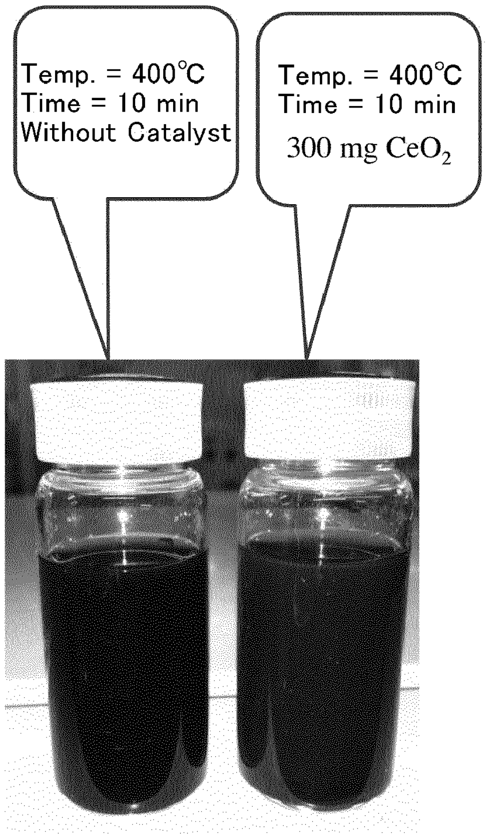
Temp. = 400°C
Time = 10 min
Without Catalyst
Temp. = 400°C
Time = 10 min
300 mg $CeO_2$

Fig.8

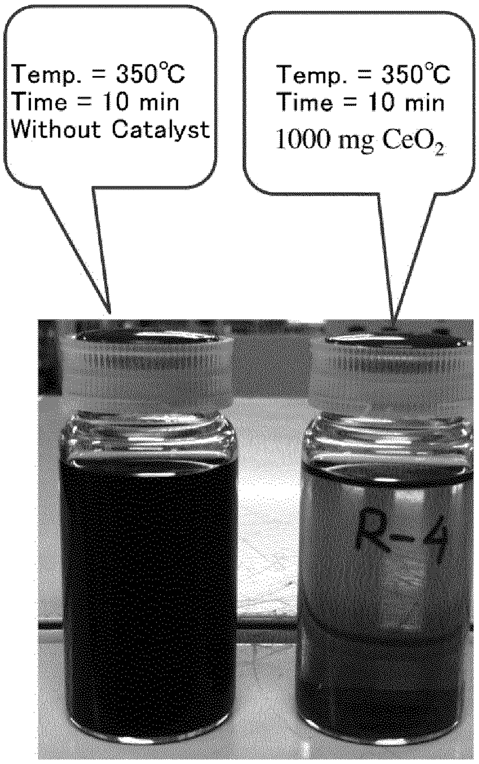
Temp. = 350℃
Time = 10 min
Without Catalyst
Temp. = 350℃
Time = 10 min
1000 mg $CeO_2$
R-4

Fig.9

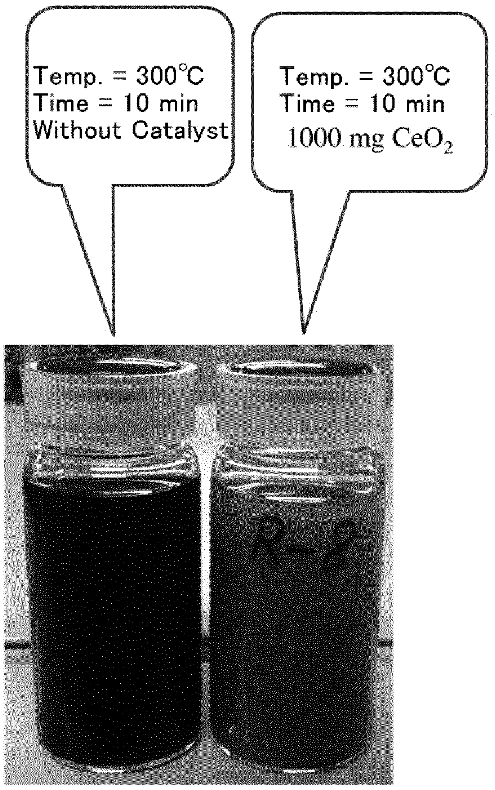
Temp. = 300℃
Time = 10 min
Without Catalyst
Temp. = 300℃
Time = 10 min
1000 mg $CeO_2$
R-8

Fig.10

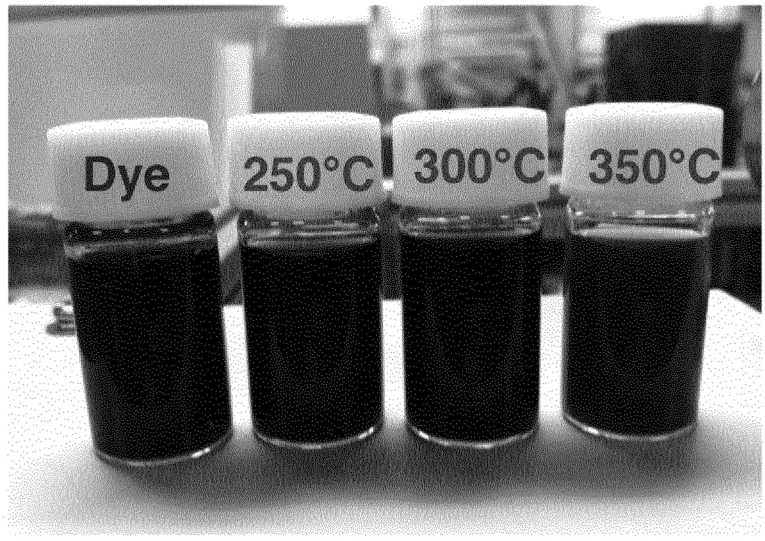


Without $CeO_2$

Fig.11

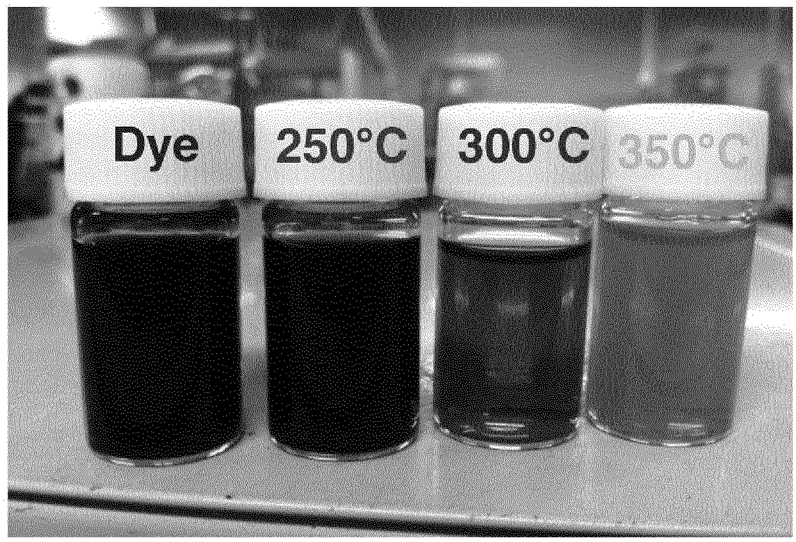


With $CeO_2$

Fig.12

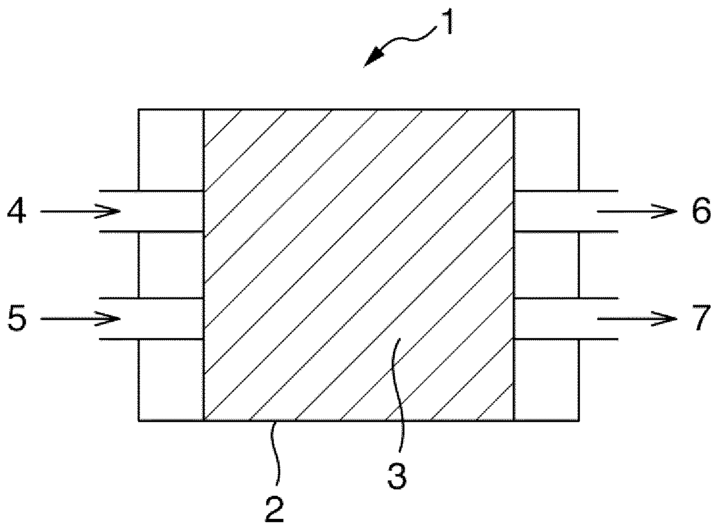


Fig.13

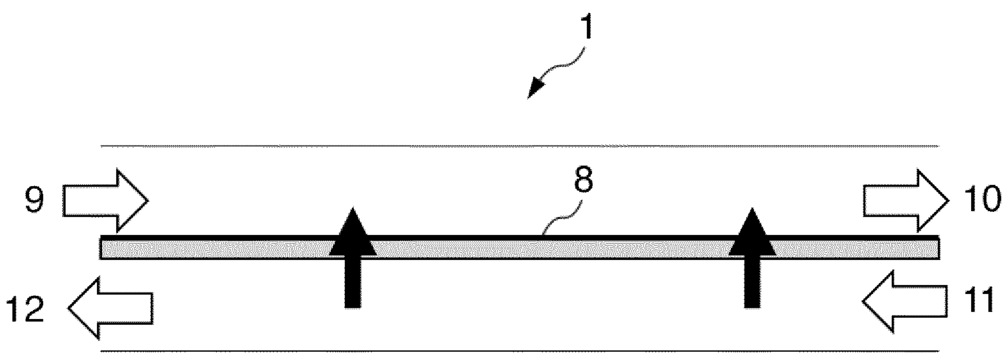

Fig.14

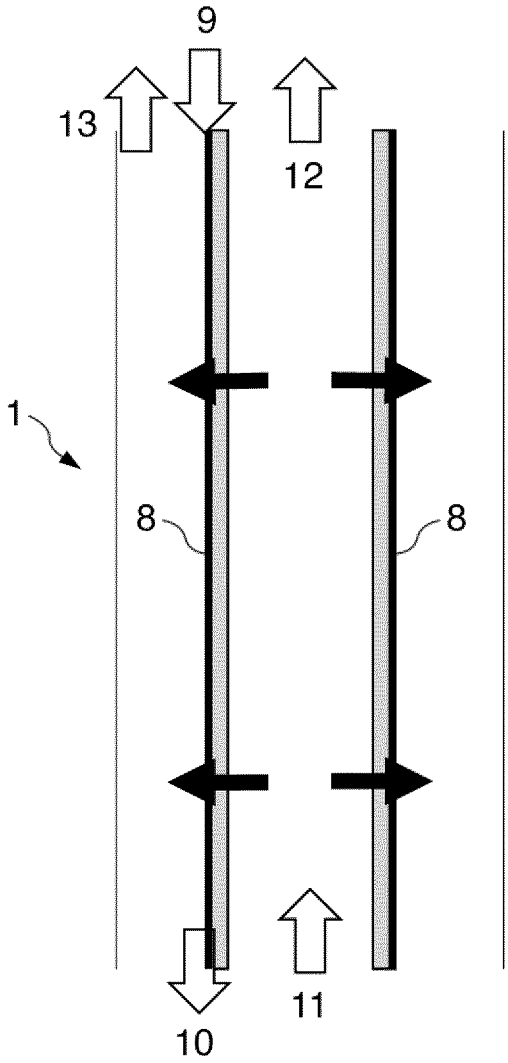

Fig.15

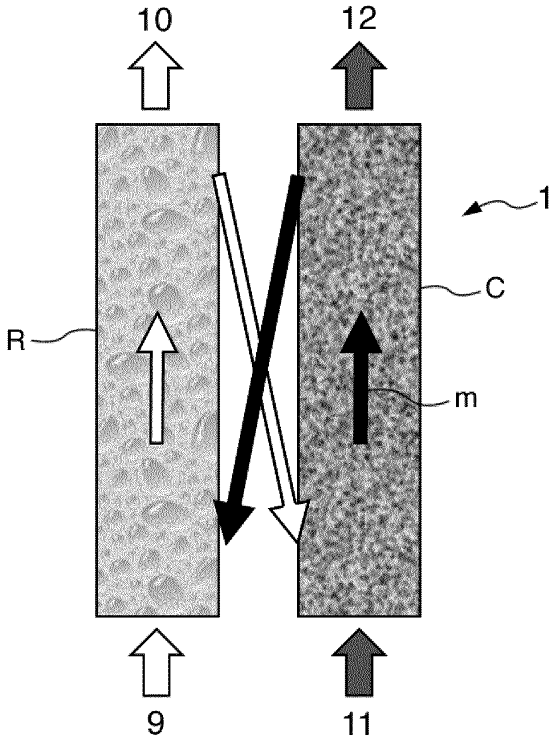


Fig.16

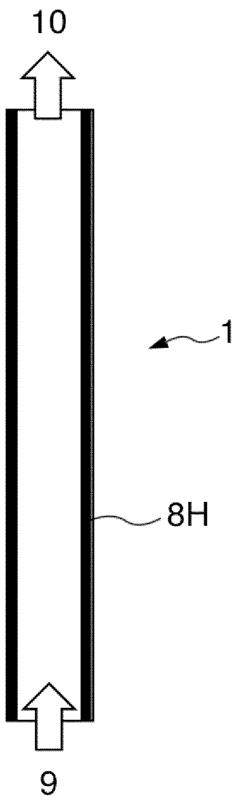

Fig.17

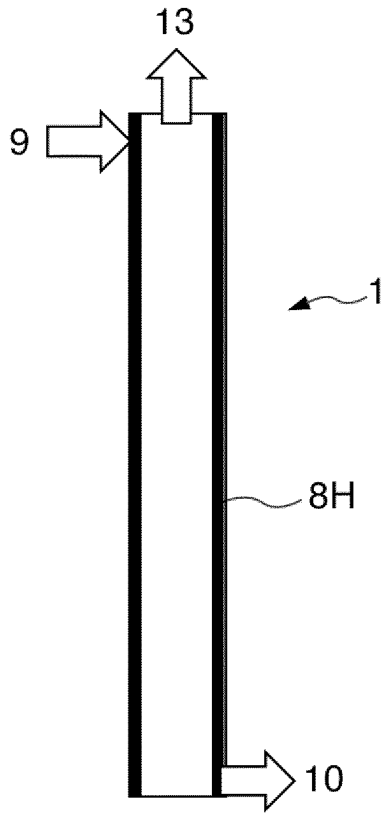


Fig.18

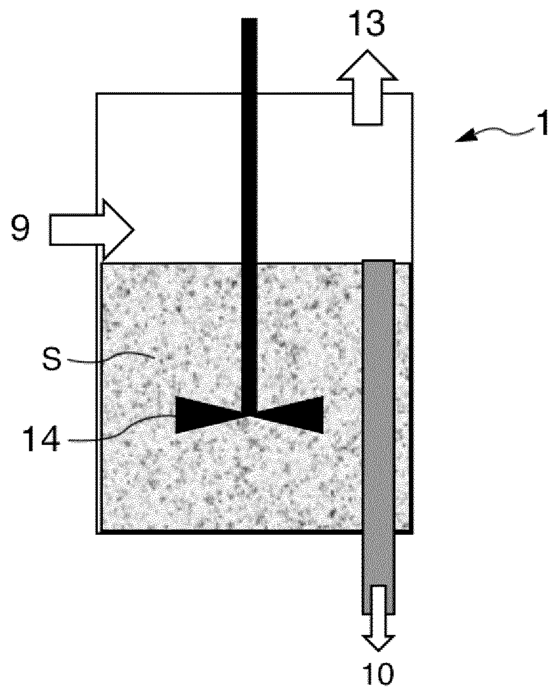

Fig.19

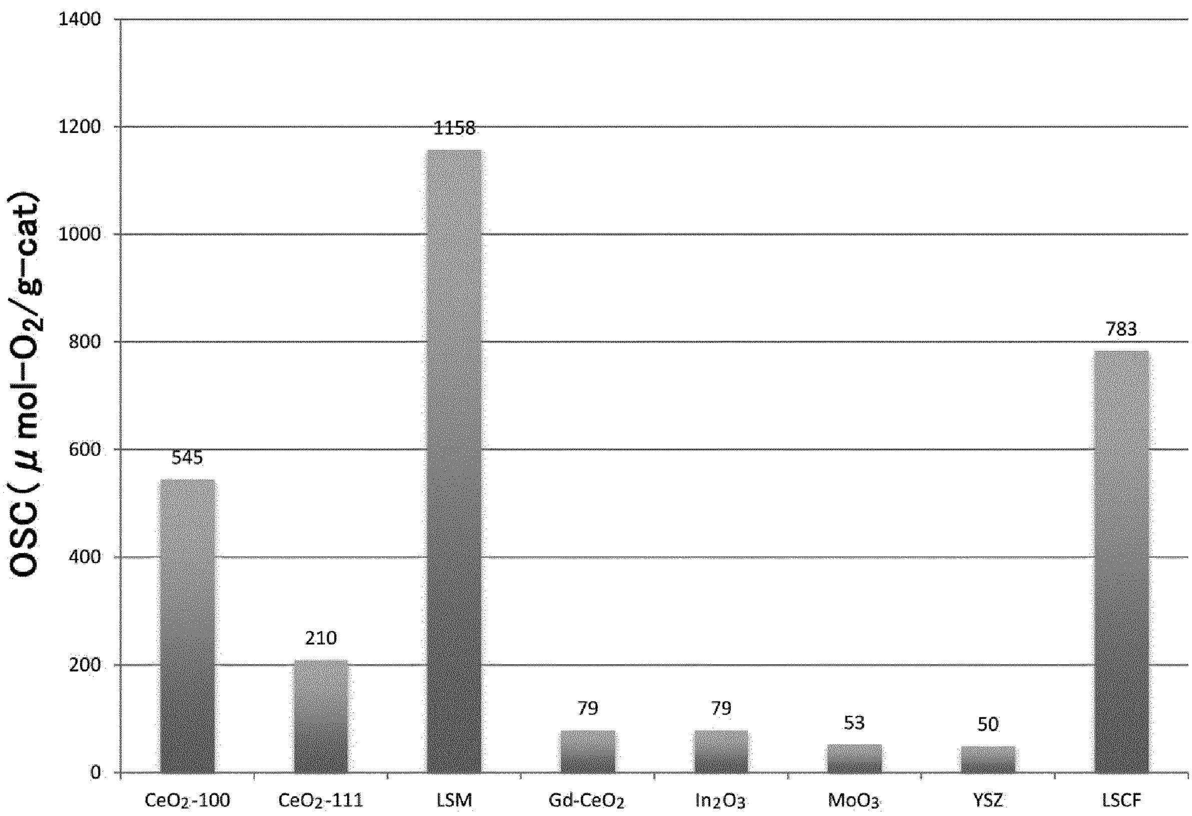
1400
1200
1000
800
600
400
200
0
OSC ( μ mol-O2/g-cat)
545
210
1158
79
79
53
50
783
CeO2-100
CeO2-111
LSM
Gd-CeO2
In2O3
MoO3
YSZ
LSCF

Fig.20

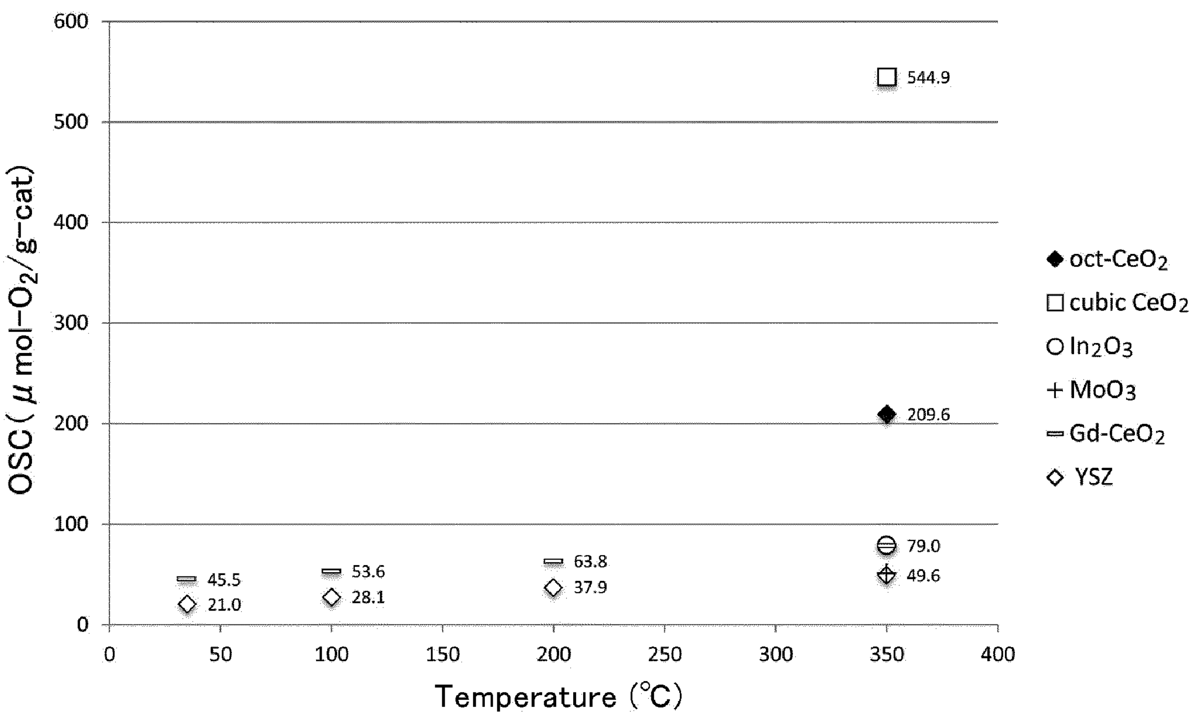
600
500
400
300
200
100
0
OSC ( μ mol-O2/g-cat)
544.9
209.6
79.0
49.6
45.5
21.0
53.6
28.1
63.8
37.9
0
50
100
150
200
250
300
350
400
Temperature (°C)
oct-CeO2
cubic CeO2
In2O3
MoO3
Gd-CeO2
YSZ

Fig.21

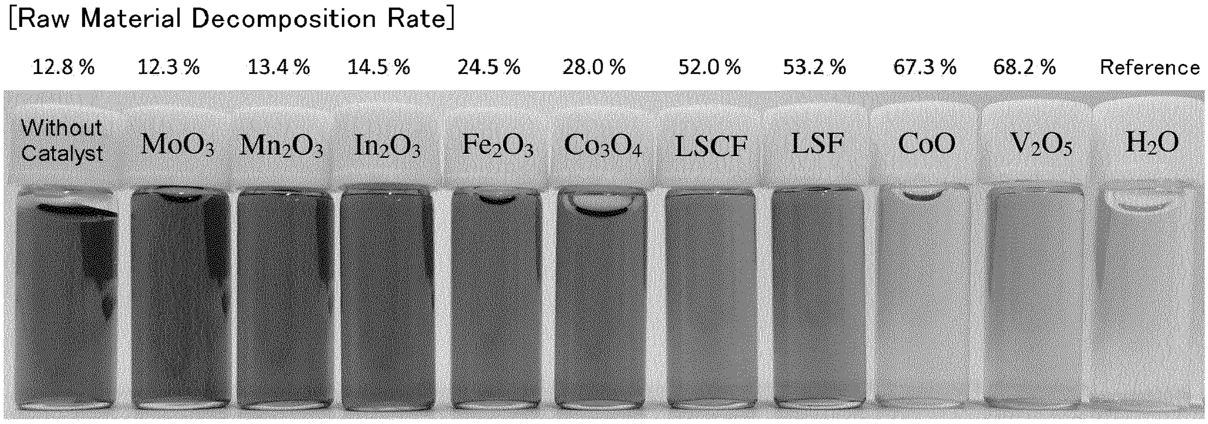
[Raw Material Decomposition Rate]
12.8 %
12.3 %
13.4 %
14.5 %
24.5 %
28.0 %
52.0 %
53.2 %
67.3 %
68.2 %
Reference
Without Catalyst
MoO3
Mn2O3
In2O3
Fe2O3
Co3O4
LSCF
LSF
CoO
V2O5
H2O

Fig.22

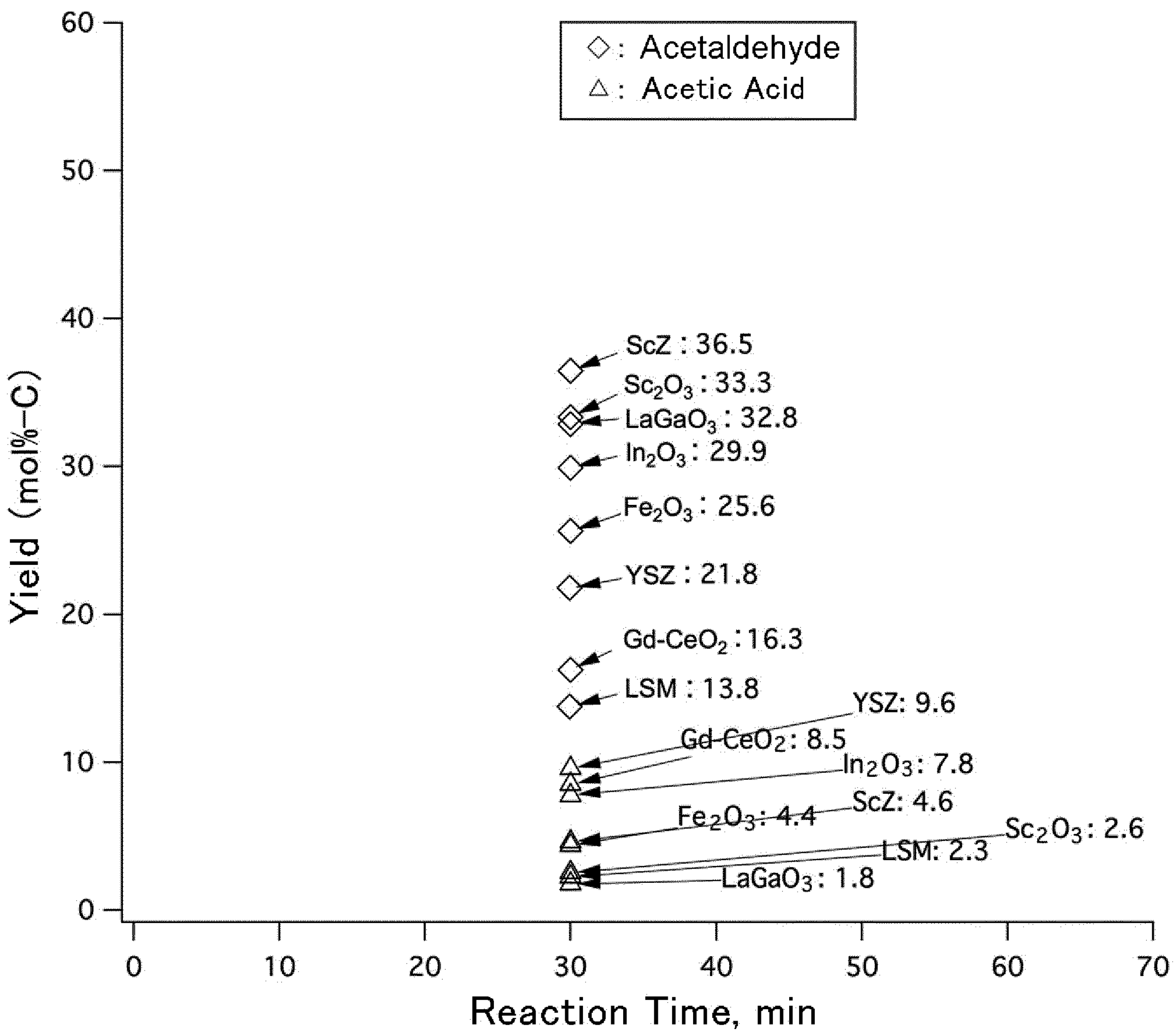
◇: Acetaldehyde
△: Acetic Acid
ScZ : 36.5
Sc2O3 : 33.3
LaGaO3 : 32.8
In2O3 : 29.9
Fe2O3 : 25.6
YSZ : 21.8
Gd-CeO2 : 16.3
LSM : 13.8
YSZ: 9.6
Gd-CeO2: 8.5
In2O3: 7.8
ScZ: 4.6
Fe2O3: 4.4
Sc2O3: 2.6
LSM: 2.3
LaGaO3: 1.8
Yield (mol%-C)
Reaction Time, min
0
10
20
30
40
50
60
70

**INTERNATIONAL SEARCH REPORT**

International application No.
PCT/JP2014/071963

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
B01J3/00, B01J23/08, B01J23/10, B01J23/22, B01J23/28, B01J23/34, B01J23/745, B01J23/75, B01J23/78, B09B3/00, C01B3/02, C01B3/40, C02F11/06, C10J3/00, C10J3/02, C10J3/46

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2012-139654 A (Ricoh Co., Ltd.), 26 July 2012 (26.07.2012), claims 1, 7, 8; paragraphs [0001], [0007], [0013], [0014], [0018] to [0030], [0049] to [0063]; fig. 1 (Family: none) | 1,5,6,9-12, 15,16,24<br>13,14,17-23, 25-30 |
| X<br>A | Medhi Dejhosseini, Tsutomu Aida, Masaru Watanabe, Seiichi Takami, Daisuke Hojo, Nobuaki Aoki, Toshihiko Arita, Atsushi Kishita, and Tadafumi Adschiri, Catalytic Cracking Reaction of Heavy Oil in the Presence of Cerium Oxide Nanoparticles in Supercritical Water, Energy & Fuels, ACS Publications, 2013.07.19, 4624-4631 | 1,5-10,12, 15,16,24<br>13,14,17-23, 25-30 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 November, 2014 (13.11.14) | 02 December, 2014 (02.12.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No. PCT/JP2014/071963

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | JP 2005-44651 A (Nissan Motor Co., Ltd.), 17 February 2005 (17.02.2005), claims 1 to 4, 6, 10; paragraphs [0001], [0021], [0023], [0024], [0026] to [0034], [0039], [0041], [0044]; fig. 1 (Family: none) | 1-6,9,10,12, 15,16,24<br>13,14,17-23, 25-30 |
| A | JP 2002-105466 A (Osaka Gas Co., Ltd.), 10 April 2002 (10.04.2002), claims 1 to 3, 5, 6; paragraphs [0001], [0002], [0009] to [0013], [0015], [0018] to [0042]; fig. 1 (Family: none) | 1-30 |
| A | JP 2001-198449 A (Komatsu Ltd.), 24 July 2001 (24.07.2001), claims 1, 3; paragraphs [0001], [0008] to [0012], [0014], [0015], [0020], [0021], [0024], [0025], [0028] to [0043], [0046], [0048], [0049], [0052], [0054]; fig. 1 to 3 (Family: none) | 1-30 |
| A | Kenji ICHIHARA, Shoji MISHIMA, Ki Chul PARK, Wataru SUGIYAMA, Hiroshi TOMIYASU, "Cho Rinkaisui-chu ni Okeru Shokubai o Mochiita Yukibutsu no Bunkai -$RuO_2$ Shokubai no Kiko to Shokubai no Saisei-", Abstracts of 36th Autumn Meeting of the Society of Chemical Engineers, Japan, The Society of Chemical Engineers, Japan, 18 August 2003 (18.08.2003), 361 | 1-30 |

Form PCT/ISA/210 (continuation of second sheet) (July 2009)

**INTERNATIONAL SEARCH REPORT**

International application No.
PCT/JP2014/071963

Continuation of A. CLASSIFICATION OF SUBJECT MATTER
(International Patent Classification (IPC))

*B01J3/00*(2006.01)i, *B01J23/08*(2006.01)i, *B01J23/10*(2006.01)i, *B01J23/22*(2006.01)i, *B01J23/28*(2006.01)i, *B01J23/34*(2006.01)i, *B01J23/745*(2006.01)i, *B01J23/75*(2006.01)i, *B01J23/78*(2006.01)i, *B09B3/00*(2006.01)i, *C01B3/02*(2006.01)i, *C01B3/40*(2006.01)i, *C02F11/06*(2006.01)i, *C10J3/00*(2006.01)i, *C10J3/02*(2006.01)i, *C10J3/46*(2006.01)i

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010144094 A
- JP 2008078334 A
- JP 6026310 A
- JP 3047110 B
- JP 3663408 B
- JP 3925936 B